# EUROPEAN PATENT SPECIFICATION

(11) **EP 3 765 631 B1**
(45) Date of publication and mention of the grant of the patent: **06.05.2026**
(21) Application number: 19767441.9
(22) Date of filing: 13.03.2019
(51) Int. Cl.: G16B 20/00, G16B 30/10

(54) **METHODS FOR THE NON-INVASIVE DETECTION AND MONITORING OF THERAPEUTIC NUCLEIC ACID CONSTRUCTS**
VERFAHREN ZUR NICHTINVASIVEN DETEKTION UND ÜBERWACHUNG THERAPEUTISCHER NUKLEINSÄUREKONSTRUKTE
MÉTHODES DE DÉTECTION ET DE SURVEILLANCE NON INVASIVES DE CONSTRUCTIONS D'ACIDES NUCLÉIQUES THÉRAPEUTIQUES

(30) Priority: 13.03.2018 US 201862642520 P; 12.04.2018 US 201862656416 P
(43) Date of publication of application: 20.01.2021
(73) Proprietor: Guardant Health, Inc., Palo Alto, CA 94304 (US)
(72) Inventor: ODEGAARD, Justin I., Redwood City, California 94063 (US); SIKORA, Marcin, Redwood City, California 94063 (US); ARTSIOMENKA, Aliaksandr, Redwood City, California 94063 (US)
(74) Representative: Carpmaels & Ransford LLP
(86) International application number: PCT/US2019/022113
(87) International publication number: WO 2019/178273

(56) References cited:
- US-A1- 2015 140 554
- US-A1- 2017 235 877
- BAOUTINA ET AL.: "Improved detection of transgene and nonviral vectors in blood", HUM GENE THER METHODS, vol. 24, no. 6, December 2013 (2013-12-01), pages 345 - 354, XP055635515
- JOHNSON ET AL.: "HybPiper: Extracting coding sequence and introns for phylogenetics from high- throughput sequencing reads using target enrichment", APPL PLANT SCI, vol. 4, no. 7, 12 July 2016 (2016-07-12), XP055635523, DOI: 10.3732/apps.1600016

## Description

### BACKGROUND

Cell-free nucleic acids (cfNA) are utilized for genomic alteration detection to inform therapy selection and is an emerging non-invasive disease monitoring tool. Therapeutic nucleic acids (TNAs) directly or indirectly manipulate DNA or mRNA transcript levels for therapeutic effect. While cfNA is in routine clinical use and TNAs are in an expanding number of clinical trials, little is known about potential interactions of these technologies, including the need for cfNA next generation sequencing (NGS) to recognize this source of exogenous DNA or RNA.

Like natural nucleic acids, nucleic acid constructs used in TNAs may be shed into the peripheral blood and may be found in the acellular fraction (i.e., not in intact cells). This material may be extracted and sequenced, and the synthetic constructs may be detected through unique synthetic sequences or over-representation of natural sequences. This detection and quantitation may be used in a number of ways, including to ascertain efficacy of administration, persistence or biological efficacy of synthetic constructs, or efficacy of therapy. However, such detection methods for TNAs in samples comprising cell-free nucleic acids are not yet established.

Baoutina et al. (Hum Gene Ther Methods. 2013; 24(6):345-54) discuss "Improved detection of transgene and nonviral vectors in blood" (title).

### SUMMARY

The invention is set out in the appended set of claims. References to methods for treatment of the human or animal body by surgery or therapy and diagnostic methods practised on the human or animal body are to be read as products, e.g. substances or compositions, for use in such methods.

In certain aspects, the present disclosure relates to a method of detecting a presence of a therapeutic nucleic acid construct in a biological sample from a test subject at least partially using a computer, the method comprising receiving, by the computer, test sequence information comprising sequence reads obtained from cell-free nucleic acid molecules in the biological sample; removing, by the computer, one or more of the sequence reads that originate from one or more intronic regions and/or from one or more regions spanning exon-intron junctions from the test sequence information to generate filtered test sequence information; and, identifying, by the computer, one or more of the sequence reads in the filtered test sequence information that substantially align with differentiating reference sequence information that originates from the therapeutic nucleic acid construct, thereby detecting the presence of the therapeutic nucleic acid construct in the biological sample from the test subject.

In certain aspects, the present disclosure relates to a method of detecting a presence of a therapeutic nucleic acid construct in a biological sample from a test subject at least partially using a computer, the method comprising receiving, by the computer, test sequence information comprising sequence reads obtained from cell-free nucleic acid molecules in the biological sample; identifying, by the computer, one or more of the sequence reads that originate from one or more regions spanning exon-exon junctions from the test sequence information to generate enriched test sequence information; and, identifying, by the computer, a presence of one or more of the sequence reads in the enriched test sequence information that substantially align with differentiating reference sequence information that originates from the therapeutic nucleic acid construct, thereby detecting the presence of the therapeutic nucleic acid construct in the biological sample from the test subject.

In certain aspects, the present disclosure relates to a method of detecting a of therapeutic nucleic acid construct in a biological sample from a test subject at least partially using a computer, the method comprising: receiving, by the computer, test sequence information comprising sequence reads obtained from cell-free nucleic acid molecules in the biological sample; identifying, by the computer, at least one set of the sequence reads comprising at least one locus in common with one another, which set comprises a coverage that exceeds a threshold value to thereby identify a candidate set of sequence reads; and, identifying, by the computer, at least some members of the candidate set of sequence reads that substantially align with reference sequence information that originates from the therapeutic nucleic acid construct, thereby detecting the presence of the therapeutic nucleic acid construct in the biological sample from the test subject.

A therapeutic nucleic acid construct can be administered to the test subject prior to receiving the test sequence information.

Monitoring therapeutic efficacy in a test subject can be performed at least partially using a computer by: receiving, by the computer, test sequence information comprising sequence reads obtained from cell-free nucleic acid molecules in biological samples from the test subject at a first time point and at a second time point, wherein a therapeutic nucleic acid construct was administered to the test subject prior to the first and/or second time point; and, quantifying, by the computer, relative amounts of the sequence reads that originate from the therapeutic nucleic acid construct, if any, at the first and second time points, thereby monitoring the therapeutic efficacy in the test subject.

Monitoring therapeutic efficacy in a test subject can be performed at least partially using a computer by: receiving, by the computer, test sequence information comprising sequence reads obtained from cell-free nucleic acid molecules in biological samples from the test subject at a first time point and at a second time point, wherein at least one control nucleic acid and at least one therapeutic nucleic acid construct were administered to the test subject prior to the first and/or second time point; and, quantifying, by the computer, relative amounts of the sequence reads that originate from the control nucleic acid and/or the therapeutic nucleic acid construct, if any, at the first and second time points, thereby monitoring the therapeutic efficacy in the test subject.

Identifying, by the computer, a size distribution among the sequence reads, can also be useful for detecting the presence of the therapeutic nucleic acid construct in the biological sample from the test subject. In certain embodiments, identifying a size distribution includes determining the presence of sequence reads with a fragment size which are typical for a therapeutic nucleic acid construct, but atypical for cell-free nucleic acid molecules. In certain embodiments, the size distribution is a unimodal size distribution. In certain embodiments, the size distribution is a bimodal size distribution.

In certain embodiments, in a sample where substantial alignments between sequence reads and differentiating reference sequence information that originates from a given therapeutic nucleic acid construct are not identified, this generally suggests, at least in the particular case, that there is at least an absence of evidence of the presence of the given therapeutic nucleic acid construct in that sample.

In certain embodiments, the methods provided herein further comprise obtaining or providing the biological sample from the subject. In certain embodiments, the biological sample is selected from the group consisting of: blood, plasma, serum, sputum, urine, semen, vaginal fluid, feces, synovial fluid, spinal fluid, mucosal excretions, sputum, tears, and saliva.

In certain embodiments, the methods provided herein further comprise generating test sequence information from the cell-free nucleic acid molecules in a biological sample. In certain embodiments, the methods provided herein further comprise amplifying one or more segments of the cell-free nucleic acid molecules in the biological sample to generate amplified nucleic acid molecules. In certain embodiments, the methods provided herein further comprise sequencing the amplified nucleic acid molecule to generate the test sequence information. In certain embodiments, the test sequence information is obtained from targeted sequences of the cell-free nucleic acid molecules in the biological sample, wherein the targeted sequences are obtained by selectively enriching one or more regions from the cell-free nucleic acid molecules in the biological sample prior to sequencing. In certain embodiments, the methods provided herein further comprise amplifying the obtained targeted sequences prior to sequencing. In certain embodiments, the sequencing is selected from the group consisting of: targeted sequencing, intron sequencing, exome sequencing, and whole genome sequencing. In certain embodiments, the methods provided herein further comprise attaching one or more adapters comprising barcodes to the cell-free nucleic acid molecules prior to sequencing.

In certain embodiments, the test subject is a mammalian subject. In certain embodiments, the mammalian subject is a human subject. In certain embodiments, the test subject has a disease or disorder capable of being treated with the therapeutic nucleic acid construct. In certain embodiments, the disease is cancer. In certain embodiments, the disorder is a genetic disorder. In certain embodiments, the disease is a viral disease. In certain embodiments, the disease or disorder is selected from the group consisting of immune deficiency disorders, hemophilia, thalassemia, sickle cell disease, blood disease, chronic granulomatous disorder, congenital blindness, lysosomal storage disease, muscular dystrophy, cancer, neurodegenerative disease, viral infections, bacterial infections, epidermolysis bullosa, heart disease, fat metabolism disorder, and diabetes, or a combination thereof.

In certain embodiments, the therapeutic nucleic acid construct is a construct used in a DNA-based therapy. In certain embodiments, the DNA-based therapeutic construct is selected from the group consisting of plasmids, aptamers, DNAzymes, antisense oligonucleotides, viral vectors, and antigene oligonucleotides. In certain embodiments, the therapeutic nucleic acid construct is a construct used in an RNA-based therapy. In certain embodiments, the RNA-based therapeutic nucleic acid construct is selected from the group consisting of aptamers, RNA decoys, antisense RNA, ribozymes, small interfering RNAs, and microRNA.

In certain embodiments, the therapeutic nucleic acid construct comprises a naturally-occurring nucleotide sequence, a variant of the naturally-occurring nucleotide sequence or a synthetic nucleotide sequence or combinations thereof. In certain embodiments, the therapeutic nucleic acid construct is part of a chimeric antigen receptor (CAR) T-cell therapy. In certain embodiments, the therapeutic nucleic acid construct is part of a CRISPR/Cas therapy. In certain embodiments, the therapeutic nucleic acid construct comprises a portion of a human adenovirus nucleic acid sequence. In certain embodiments, the biological sample comprises fragments of the therapeutic nucleic acid construct.

In certain embodiments, the methods provided herein further comprise selectively enriching for a target sequence of the therapeutic nucleic acid construct. In certain embodiments, the target sequence comprises a gene or a variant thereof selected from the group consisting of *TP53, HBB, RPE65, and B-domain deleted factor VIII gene, DMD, UL123, APOB, SMN1, SMN2, ICAM-1, TLR9, IRS-1, VEGF, PDGFA, PDGFB, PDGFC, PDGFD,* and *TTR.*

In certain aspects, the present disclosure relates to a system, comprising a controller comprising, or capable of accessing, computer readable media comprising non-transitory computer-executable instructions which, when executed by at least one electronic processor perform at least: receiving test sequence information comprising sequence reads obtained from cell-free nucleic acid molecules in a biological sample from a test subject; removing one or more of the sequence reads that originate from one or more intronic regions and/or from one or more regions spanning exon-intron junctions from the test sequence information to generate filtered test sequence information; and, identifying one or more of the sequence reads in the filtered test sequence information that substantially align with differentiating reference sequence information that originates from a therapeutic nucleic acid construct.

In certain aspects, the present disclosure relates to a system, comprising a controller comprising, or capable of accessing, computer readable media comprising non-transitory computer-executable instructions which, when executed by at least one electronic processor perform at least: receiving test sequence information comprising sequence reads obtained from cell-free nucleic acid molecules in a biological sample from a test subject; identifying one or more of the sequence reads that originate from one or more regions spanning exon-exon junctions from the test sequence information to generate enriched test sequence information; and, identifying one or more of the sequence reads in the enriched test sequence information that substantially align with differentiating reference sequence information that originates from a therapeutic nucleic acid construct.

In certain aspects, the present disclosure relates to a system, comprising a controller comprising, or capable of accessing, computer readable media comprising non-transitory computer-executable instructions which, when executed by at least one electronic processor perform at least: receiving test sequence information comprising sequence reads obtained from cell-free nucleic acid molecules in a biological sample from a test subject; identifying at least one set of the sequence reads comprising at least one locus in common with one another, which set comprises a coverage that exceeds a threshold value to thereby identify a candidate set of sequence reads; and, identifying at least some members of the candidate set of sequence reads that substantially align with reference sequence information that originates from a therapeutic nucleic acid construct.

In certain embodiments, the system disclosed herein, comprises a nucleic acid sequencer operably connected to the controller, which nucleic acid sequencer is configured to provide the test sequence information from the cell-free nucleic acid molecules in the biological sample. In certain embodiments, the nucleic acid sequencer is configured to perform pyrosequencing, single-molecule sequencing, nanopore sequencing, semiconductor sequencing, sequencing-by-ligation or sequencing-by-hybridization on the cell-free nucleic acid molecules to generate the sequencing reads. In certain embodiments, a sample preparation component is operably connected to the controller, which sample preparation component is configured to prepare the cell-free nucleic acid molecules to be sequenced by a nucleic acid sequencer. In certain embodiments, the sample preparation component is configured to selectively enrich regions from the cell-free nucleic acid molecules in the biological sample. In certain embodiments, the sample preparation component is configured to attach one or more adapters comprising barcodes to the cell-free nucleic acid molecules.

In certain embodiments, the system comprises a nucleic acid amplification component operably connected to the controller, which nucleic acid amplification component is configured to amplify the cell-free nucleic acid molecules. In certain embodiments, the nucleic acid amplification component is configured to amplify selectively enriched regions from the cell-free nucleic acid molecules in the biological DNA sample.

In certain embodiments, the system comprises a material transfer component operably connected to the controller, which material transfer component is configured to transfer one or more materials between a nucleic acid sequencer and a sample preparation component.

In certain aspects, the present disclosure relates to a computer readable media comprising non-transitory computer-executable instructions which, when executed by at least one electronic processor perform at least: receiving test sequence information comprising sequence reads obtained from cell-free nucleic acid molecules in a biological sample from a test subject; removing one or more of the sequence reads that originate from one or more intronic regions and/or from one or more regions spanning exon-intron junctions from the test sequence information to generate filtered test sequence information; and, identifying one or more of the sequence reads in the filtered test sequence information that substantially align with differentiating reference sequence information that originates from a therapeutic nucleic acid construct.

In certain aspects, the present disclosure relates to a computer readable media comprising non-transitory computer-executable instructions which, when executed by at least one electronic processor perform at least: receiving test sequence information comprising sequence reads obtained from cell-free nucleic acid molecules in a biological sample from a test subject; identifying one or more of the sequence reads that originate from one or more regions spanning exon-exon junctions from the test sequence information to generate enriched test sequence information; and, identifying one or more of the sequence reads in the enriched test sequence information that substantially align with differentiating reference sequence information that originates from a therapeutic nucleic acid construct.

In certain aspects, the present disclosure relates to a computer readable media comprising non-transitory computer-executable instructions which, when executed by at least one electronic processor perform at least: receiving test sequence information comprising sequence reads obtained from cell-free nucleic acid molecules in a biological sample from a test subject; identifying at least one set of the sequence reads comprising at least one locus in common with one another, which set comprises a coverage that exceeds a threshold value to thereby identify a candidate set of sequence reads; and, identifying at least some members of the candidate set of sequence reads that substantially align with reference sequence information that originates from a therapeutic nucleic acid construct.

In certain embodiments, the computer readable media further comprises non-transitory computer-executable instructions which, when executed by the at least one electronic processor perform at least: causing a sample preparation component operably connected to the electronic processor to prepare the cell-free nucleic acid molecules to be amplified and/or sequenced; causing a nucleic acid amplification component operably connected to the electronic processor to amplify the cell-free nucleic acid molecules; causing a nucleic acid sequencer operably connected to the electronic processor to sequence the cell-free nucleic acid molecules; and, causing a material transfer component operably connected to the electronic processor to transfer one or more materials between the sample preparation component, the nucleic acid amplification component, and/or the nucleic acid sequencer.

In certain embodiments, the computer readable media further comprises non-transitory computer-executable instructions which, when executed by the at least one electronic processor perform at least: aligning one or more of the sequence reads with reference sequence information, wherein one or more databases, operably connected to the electronic processor, comprise the reference sequence information. In certain embodiments, the computer readable media further comprises non-transitory computer-executable instructions which, when executed by the at least one electronic processor perform at least: display one or more of the sequence reads, and/or data derived therefrom, that originate from the therapeutic nucleic acid construct on at least one display that is operably connected to the electronic processor.

A method for treating a subject can comprise: receiving, by a computer, test sequence information comprising sequence reads obtained from cell-free nucleic acid molecules in biological samples from the subject at a first time point and at a second time point, wherein a therapeutic nucleic acid construct was administered to the test subject prior to the first and/or second time point; quantifying, by the computer, relative amounts of the sequence reads that originate from the therapeutic nucleic acid construct, if any, at the first and second time point; and administering a further dosage of the therapeutic nucleic acid construct to the subject if the amount of sequence reads that originate from the therapeutic nucleic acid construct is below a threshold at the second time point.

A method for treating a subject can comprise: receiving, by a computer, test sequence information comprising sequence reads obtained from cell-free nucleic acid molecules in biological samples from the subject at a first time point and at a second time point, wherein a therapeutic nucleic acid construct was administered to the test subject prior to the first and/or second time point; quantifying, by the computer, relative amounts of the sequence reads that originate from the therapeutic nucleic acid construct, if any, at the first and second time point; and administering a further dosage of the therapeutic nucleic acid construct to the subject if the amount of sequence reads that originate from the therapeutic nucleic acid construct is below a threshold at the second time point.

A method for treating a subject can comprise: receiving, by a computer, test sequence information comprising sequence reads obtained from cell-free nucleic acid molecules in biological samples from the subject at a first time point and at a second time point, wherein at least one control nucleic acid and at least one therapeutic nucleic acid construct were administered to the test subject prior to the first and/or second time point; quantifying, by the computer, relative amounts of the sequence reads that originate from the control nucleic acid and/or the therapeutic nucleic acid construct, if any, at the first and second time points; and administering a further dosage of the therapeutic nucleic acid construct to the subject if the amount of sequence reads that originate from the control nucleic acid and/or therapeutic nucleic acid construct is below a threshold at the second time point.

A composition comprising a control nucleic acid and a therapeutic nucleic acid construct can be used in a method of monitoring therapeutic efficacy of the therapeutic nucleic acid construct in a test subject, the method comprising: receiving, by a computer, test sequence information comprising sequence reads obtained from cell-free nucleic acid molecules in biological samples from the test subject at a first time point and at a second time point, wherein the composition was administered to the test subject prior to the first and/or second time point; and, quantifying, by the computer, relative amounts of the sequence reads that originate from the control nucleic acid and/or the therapeutic nucleic acid construct, if any, at the first and second time points, thereby monitoring the therapeutic efficacy in the test subject.

### BRIEF DESCRIPTION OF THE FIGURES

FIG. 1 shows an embodiment of a therapeutic nucleic acid construct comprising a liposome-encapsulated TP53 expression vector.
FIG. 2 shows a graph indicating an increase in coverage amplification for TP53 on chromosome 17 compared to a baseline measurement.
FIG. 3 shows a diagram of mapped sequences reads of TP53. A portion of the sequence reads do not map to introns of the reference sequence, indicating intron deletion.
FIG. 4A and FIG. 4B show a diagram of mapped and unmapped sequence reads of the TP53 expression vector used for detection of the therapeutic nucleic acid construct. In FIG. 4A, an unmapped sequence read (foreign sequence) is determined to be located adjacent to TP53 exon 1. In FIG 4B, an unmapped sequence read (foreign sequence) is determined to be located adjacent to TP53 exon 11.
FIG. 5 shows a unimodal size distribution graph of the TP53 therapeutic nucleic acid construct (dotted line) compared to the length of cfDNA molecules of cellular origin (solid line).
FIG. 6 is a schematic diagram of an exemplary system suitable for use with certain embodiments.
FIG. 7 shows an assembly workflow of a foreign TP53-flanking sequence into a 6003 bp circular construct.

### DEFINITIONS

While various embodiments of the disclosure have been shown and described herein, those skilled in the art will understand that such embodiments are provided by way of example only.

The term "subject" or "patient" refers to an animal, such as a mammalian species (preferably human) or avian (e.g., bird) species, or other organisms. More specifically, a subject can be a vertebrate, e.g., a mammal such as a mouse, a primate, a simian or a human. Animals include farm animals, sport animals, and pets. A subject can be a healthy individual, an individual that has symptoms or signs or is suspected of having a disease or a predisposition to the disease, or an individual that is in need of therapy or suspected of needing therapy. In some embodiments, the subject is human such as a human who has, or is suspected of having cancer.

The phrase "cell-free nucleic acid" or "cfNA" refers to nucleic acids not contained within or otherwise bound to a cell or in other words nucleic acids remaining in a sample after removing intact cells. Cell-free nucleic acids can be referred to as all non-encapsulated nucleic acid sourced from a bodily fluid (e.g., blood, urine, CSF, etc.) from a subject. Cell-free nucleic acids include DNA (cfDNA), RNA (cfRNA), and hybrids thereof, including genomic DNA, mitochondrial DNA, circulating DNA, siRNA, miRNA, circulating RNA (cRNA), tRNA, rRNA, small nucleolar RNA (snoRNA), Piwi-interacting RNA (piRNA), long non-coding RNA (long ncRNA), or fragments of any of these. Cell-free nucleic acids can be double-stranded, single-stranded, or partially double- and single-stranded. A cell-free nucleic acid can be released into bodily fluid through secretion or cell death processes, e.g., cellular necrosis and apoptosis. Some cell-free nucleic acids are released into bodily fluid from cancer cells, e.g., circulating tumor DNA (ctDNA). Others are released from healthy cells. ctDNA can be non-encapsulated tumor-derived fragmented DNA. Cell-free fetal DNA (cffDNA) is fetal DNA circulating freely in the maternal blood stream. A cell-free nucleic acid can have one or more associated epigenetic modifications, for example, acetylation, 5-methylatation, ubiquitylation, phosphorylation, sumoylation, ribosylation, and/or citrullination. In some embodiments, cell-free nucleic acid is cfDNA, which usually includes double-stranded cfDNA.

The term "tag" refers to a short nucleic acid (e.g., less than 500, 100, 50 or 10 nucleotides long), used to label nucleic acid molecules to distinguish nucleic acids from different samples (e.g., representing a sample index), or different nucleic acid molecules in the same sample (e.g., representing a molecular barcode), of different types, or which have undergone different processing. Tags can be single stranded, double-stranded or at least partially double-stranded. Tags can have the same length or varied lengths. Tags can be blunt-ended or have an overhang. Tags can be attached to one end or both ends of the nucleic acids. Tags can be decoded to reveal information such as the sample of origin, type (e.g., RNA or DNA, strand orientation, etc.) or processing of a nucleic acid. Tags can be used to allow pooling and parallel processing of multiple samples comprising nucleic acids bearing different molecular barcodes and/or sample indexes with the nucleic acids subsequently being deconvoluted by reading the tags. Additionally or alternatively, tags can be used to distinguish different molecules in the same sample. This includes using molecular barcodes to uniquely barcode different molecules in the sample, or non-uniquely barcode molecules. In the case of non-unique barcoding, a limited number of different barcodes may be used to tag molecules such that different molecules can be distinguished based on their start/stop position where they map on a reference genome in combination with at least one barcode. Typically then, a sufficient number of different tags are used such that there is a low probability (e.g. <10%, < 5%, <1%, or <0.1%) that any two molecules having the same start/stop also have the same barcode. Some tags include multiple molecular identifiers to label samples, forms of molecule within a sample, and molecules within a form having the same start and stop points. Such tags can exist in the form A1i, wherein the letter indicates a sample type, the Arabic number indicates a form of molecule within a sample, and the Roman numeral indicates a molecule within a form.

The term "adapter" refers to a short nucleic acid (e.g., less than 500, 100 or 50 nucleotides long) usually at least partly double-stranded for linkage to either or both ends of a sample nucleic acid molecule. Adapters can include primer binding sites to permit amplification of a nucleic acid molecule flanked by adapters at both ends, and/or a sequencing primer binding site, including primer binding sites for next generation sequencing (NGS). Adapters can also include binding sites for capture probes, such as an oligonucleotide attached to a flow cell support. Adapters can also include a tag as described above. Tags are preferably positioned relative to primer and sequencing primer binding sites, such that a tag is included in amplicons and sequencing reads of a nucleic acid molecule. Adapters having the same or different nucleotide sequences can be linked to the respective ends of a nucleic acid molecule. Sometimes adapters of the same sequence are linked to the respective ends except that the tag is different. A preferred adapter is a Y-shaped adapter in which one end is blunt ended or tailed as described herein, for joining to a nucleic acid molecule, which is also blunt ended or tailed with one or more complementary nucleotides. Another preferred adapter is a bell-shaped adapter, likewise with a blunt or tailed end for joining to a nucleic acid to be analyzed. Other exemplary adapters include T-tailed and C-tailed adapters.

As used herein, the terms "sequencing" or "sequencer" refer to any of a number of technologies used to determine the sequence of a biomolecule, e.g., a nucleic acid such as DNA or RNA. Exemplary sequencing methods include, but are not limited to, targeted sequencing, single molecule real-time sequencing, exon sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{™} sequencing, MS-PET sequencing, and a combination thereof. In some embodiments, sequencing can be performed by a gene analyzer such as, for example, gene analyzers commercially available from Illumina or Applied Biosystems.

The phrase "next generation sequencing" or NGS refers to sequencing technologies having increased throughput as compared to traditional Sanger- and capillary electrophoresis-based approaches, for example, with the ability to generate hundreds of thousands of relatively small sequence reads at a time. Some examples of next generation sequencing techniques include, but are not limited to, sequencing by synthesis, sequencing by ligation, and sequencing by hybridization.

The term "DNA" (deoxyribonucleic acid) refers to a chain of nucleotides comprising deoxyribonucleosides that each comprise one of four nucleobases, namely, adenine (A), thymine (T), cytosine (C), or guanine (G). The term "RNA (ribonucleic acid)" refers to a chain of nucleotides comprising four types of ribonucleosides that each comprise one of four nucleobases, namely; A, uracil (U), G, or C. Certain pairs of nucleotides specifically bind to one another in a complementary fashion (called complementary base pairing). In DNA, adenine (A) pairs with thymine (T) and cytosine (C) pairs with guanine (G). In RNA, adenine (A) pairs with uracil (U) and cytosine (C) pairs with guanine (G). When a first nucleic acid strand binds to a second nucleic acid strand made up of nucleotides that are complementary to those in the first strand, the two strands bind to form a double strand. As used herein, "nucleic acid sequencing data," "nucleic acid sequencing information," "nucleic acid sequence," "nucleotide sequence," "genomic sequence," "genetic sequence," "sequence read," "fragment sequence," or "nucleic acid sequencing read" denotes any information or data that is indicative of the order of the nucleotide bases (e.g., adenine, guanine, cytosine, and thymine or uracil) in a molecule (e.g., a whole genome, whole transcriptome, exome, oligonucleotide, polynucleotide, or fragment) of a nucleic acid such as DNA or RNA. It should be understood that the present teachings contemplate sequence information obtained using all available varieties of techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, and electronic signature-based systems.

A "polynucleotide", "nucleic acid", "nucleic acid molecule", or "oligonucleotide" refers to a linear polymer of nucleosides (including deoxyribonucleosides, ribonucleosides, or analogs thereof) joined by internucleosidic linkages. Typically, a polynucleotide comprises at least three nucleosides. Oligonucleotides often range in size from a few monomeric units, e.g. 3-4, to hundreds of monomeric units. Whenever a polynucleotide is represented by a sequence of letters, such as "ATGCCTG," it will be understood that the nucleotides are in 5' → 3' order from left to right and that "A" denotes deoxyadenosine, "C" denotes deoxycytidine, "G" denotes deoxyguanosine, and "T" denotes thymidine, unless otherwise noted. The letters A, C, G, and T may be used to refer to the bases themselves, to nucleosides, or to nucleotides comprising the bases, as is standard in the art.

The phrase "reference sequence" refers to a known sequence used for purposes of comparison with experimentally determined sequences. For example, a known sequence can be an entire genome, a chromosome, a gene, a therapeutic nucleic acid construct, or any segment thereof. A reference typically includes at least 20, 50, 100, 200, 250, 300, 350, 400, 450, 500, 1000, or more nucleotides. A reference sequence can align with a single contiguous sequence of a genome or chromosome or can include non-contiguous segments aligning with different regions of a genome or chromosome. In some embodiments, the reference sequence is a human genome. Reference human genomes include, e.g., hG19 and hG38. In some embodiments, the reference sequence is a known, or partially known, sequence for which at least a portion of the therapeutic nucleic acid construct aligns to. This may include, in some cases, a portion of a therapeutic nucleic acid construct comprising a sequence that aligns to the human genome. In other cases, this may include a portion of a therapeutic nucleic acid construct comprising a sequence that aligns to a known or partially known sequence other than a sequence of the human genome (e.g., a promoter sequence of a vector aligning to a reference sequence comprising a portion of the promoter sequence). In some embodiments, more than one reference sequence is used to detect the therapeutic nucleic acid construct.

The phrase "gene therapy" refers to a treatment of a subject's body or isolated elements of a subject's body, for example isolated tissues/cells, by a therapeutic nucleic acid construct comprising nucleic acids, some of which may encode for a peptide or protein. It typically may comprise at least one of the steps of a) administration of a therapeutic nucleic acid construct directly to the patient by whatever administration route (as discussed herein) or *in vitro* to isolated cells/tissues of the subject, which results in transfection of the subject's cells either *in vivo*/*ex vivo* or *in vitro,* b) transcription and/or translation of the introduced therapeutic nucleic acid construct; and optionally c) re-administration of isolated, transfected cells to the subject, if the therapeutic nucleic acid construct has not been administered directly to the subject.

The phrase "therapeutic nucleic acid construct" refers to a polymer comprising deoxyribonucleotides, ribonucleotides, and/or modified or analog nucleotides that is used to treat a disease, disorder, or condition. Modified or analog nucleotides used in therapeutic nucleic acid constructs typically include modified base, modified sugar, and/or modified phosphate moieties. Some examples of modified or analog nucleotides include methyl phosphonates, phosphorothioates, phosphoramidates, peptide-nucleic acids (PNAs), chiral-methyl phosphonates, 2-O-methyl ribonucleotides, locked nucleic acids (LNAs), and/or the like. Exemplary therapeutic nucleic acid constructs include plasmids, aptamers, DNAzymes, antisense oligonucleotides (ASOs), viral vectors, antigene oligonucleotides, aptamers, RNA decoys, antisense RNA, ribozymes, small interfering RNAs, microRNA, gRNA, crRNA, tracrRNA, and the like. In some embodiments, therapeutic nucleic acid constructs are encapsulated in cells, liposomes or other lipid containing particles, viral vectors, and the like, for administration to subjects.

The phrase "biological sample" as used herein, generally refers to a tissue or fluid sample derived from a subject. A biological sample may be directly obtained from the subject. The biological sample may be or may include one or more nucleic acid molecules, such as deoxyribonucleic acid (DNA) or ribonucleic acid (RNA) molecules. The biological sample can be derived from any organ, tissue or biological fluid. A biological sample can comprise, for example, a bodily fluid or a solid tissue sample. An example of a solid tissue sample is a tumor sample, e.g., from a solid tumor biopsy. Bodily fluids include, for example, blood, serum, plasma, tumor cells, saliva, urine, lymphatic fluid, prostatic fluid, seminal fluid, milk, sputum, stool, tears, and derivatives of these. In some embodiments, the biological sample is, or is derived from, blood.

The phrase "TP53 gene" refers to a gene that codes for a protein (EC:2.7.1.37) that regulates the cell cycle and is involved in tumor suppression. The human p53 gene is located on the seventeenth chromosome (17p13.1).

The phrase "substantially align" in the context of nucleic acid sequence alignment means that a first nucleic acid sequence has at least 95%, at least 96%, at least 97%, at least 98%, at least 99%, or even 100% sequence identity with at least a sub-sequence of a second nucleic acid sequence. In some embodiments, for example, a given sequence read substantially aligns with a reference sequence when the given sequence read has 95%, 96%, 97%, 98%, 99%, or 100% sequence identity with at least a sub-sequence or region, or the entirety, of the reference sequence.

The phrase "differentiating reference sequence information" in the context of therapeutic nucleic acid constructs means one or more sequence regions of a given therapeutic nucleic acid construct that differentiate or distinguish that given therapeutic nucleic acid construct from other nucleic acid sequences observed in a given biological sample from a given subject. In certain embodiments, the differentiating reference sequence information may include sequences of the therapeutic nucleic acid construct that have no similarity to the human genome (herein sometimes referred to as "foreign sequences" or "exogenous nucleic acid molecules"). In certain embodiments, the differentiating reference sequence information may include sequences of the therapeutic nucleic acid construct that have partial similarity to the human genome (e.g., segments of a human gene sequence with the introns deleted).

The phrase "administer" in the context of therapeutic agents (e.g., therapeutic nucleic acid constructs) means to give, apply or bring the agents into contact with a subject. Administration can be accomplished by any of a number of routes, including, for example, topical, oral, subcutaneous, intramuscular, intraperitoneal, intravenous, intrathecal and intradermal.

The phrase "about" or "approximately" as applied to one or more values or elements of interest, refers to a value or element that is similar to a stated reference value or element. In certain embodiments, the term "about" or "approximately" refers to a range of values or elements that falls within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value or element unless otherwise stated or otherwise evident from the context (except where such number would exceed 100% of a possible value or element).

### DETAILED DESCRIPTION

### I. General Overview

Recent approval by the U.S. Food and Drug Administration of new gene therapies is a turning point in the field of genetic medicine. With many other gene therapies in clinical trials, the ability to detect and monitor therapeutic nucleic acid constructs used in these treatments will be paramount for safety and efficacy purposes going forward. Accordingly, the methods, systems and compositions disclosed herein provide a non-invasive way for detecting therapeutic nucleic acid constructs and monitoring the efficacy of these constructs in samples comprising cell-free nucleic acid from a patient.

A therapeutic nucleic acid construct is a polymer comprising deoxyribonucleotides, ribonucleotides, and/or modified or analog nucleotides that is used to treat a disease, disorder, or condition. The composition of the construct may vary depending on the type of therapy for which it is designed. For example, a construct may be used in DNA-based therapies, and thus, may include, but not be limited to, DNA aptamers, plasmids, viral vectors, DNAzymes, antisense oligonucleotides, and antigene oligonucleotides. In other cases, a construct may be used in RNA-based therapies, and may include, but not be limited to, RNA aptamers, RNA decoys, antisense RNA, ribozymes, small interfering RNAs, RNA interference, microRNA, or circular RNAs. In some embodiments where therapeutic nucleic acid constructs may consist of sequences that are indistinguishable from at least portions of a subject's genome (e.g., as is the case of certain antisense constructs, including certain antisense RNA constructs and the like), detection of the constructs according to the methods of the present disclosure is via over-representation (e.g., an atypical abundance) of sequence reads observed in a given sample that map to or substantially align with those sequences in certain embodiments.

A therapeutic nucleic acid construct may be used in a gene therapy, including germline or somatic therapies. In some cases, the therapy may comprise a viral therapy, for example, Imlygic^{™} or Luxturna. In some cases, the therapy may comprise antisense oligonucleotides, for example, formivirsen, mipomersen, eteplirsen, nusinersen, aicaforsen, aganirsen, and the like. In some cases, the therapy may comprise an aptamer, for example, pegaptanib or pegpleranib. In some cases, the therapy may comprise siRNA, for example, patisiran and QPI-1002.

In some cases, the therapy may comprise a chimeric antigen receptor (CAR) T-cell therapy, for example, Kymriah^{™} or Yescarta^{™}. In certain embodiments, therapeutic nucleic acid constructs include at least portions of vectors (e.g., gammaretrovirus (RV), lentivirus (LV), or other suitable vectors) used as part of the CAR-T cell therapeutic processes. In some embodiments, the therapeutic nucleic acid construct may be part of a gene editing therapy, such as in a CRISPR-Cas therapy (e.g., CRISPR-Cas3 or CRISPR-Cas9 complex).

Therapeutic nucleic acids may be administered to a subject in any manner known in the art. Such methods include, but are not limited to viral delivery (e.g., retroviruses, adenoviruses, pseudotyped viruses, etc.) or non-viral delivery (e.g., injection, electroporation, sonoporation, magnetofection, nanoparticles, lipoplexes, eye droplets, etc.). Administration may occur via any known route, for example, *in vivo, ex vivo, in vitro* or *in situ* delivery.

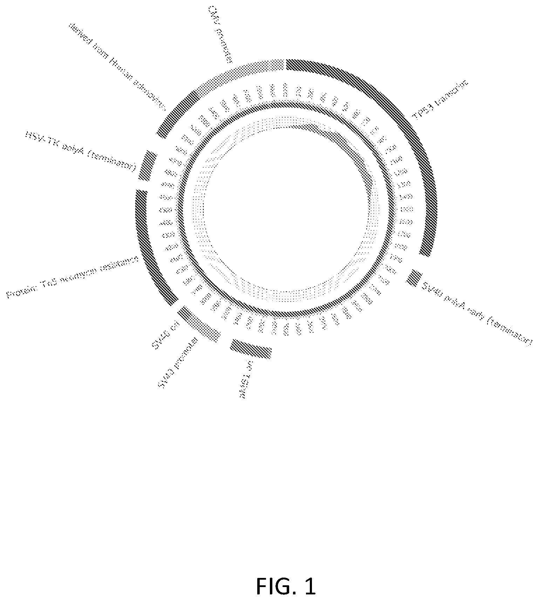

A representative, non-limiting example of a therapeutic nucleic acid construct is shown in FIG. 1. Referring to this figure, the construct is a 6003 bp circular liposome-encapsulated TP53 expression vector comprising origins of replication sequences (pMB1 ori and SV40 ori), an SV40 promoter, a Tn5 neomycin resistance sequence, HSV-TK polyA terminator sequence, a sequence derived from human adenovirus, a CMV promotor sequence, the TP53 cDNA or gene sequence, and an SV40 polyA terminator sequence. The inner circular graph in FIG. 1 shows sequencing coverage of different components of the construct.

The methods, systems, and compositions may be particularly useful in the analysis of cell-free nucleic acid molecules. In some cases, cell-free nucleic acid molecules may be extracted and isolated from a biological sample from a subject. A biological sample may include a bodily fluid sample that is selected from the group including, but not limited to blood, plasma, serum, urine, saliva, mucosal excretions, sputum, stool, and tears. Cell-free nucleic acid molecules can be extracted using a variety of methods known in the art, including but not limited to isopropanol precipitation and/or silica based purification.

The biological sample may be collected from a number of subjects, such as subjects without a disease, subjects at risk for, showing symptoms of, or having a disease, such as cancer or a viral infection, or subjects at risk for, showing symptoms of, or having a genetic disorder. In some embodiments, the disease or disorder is selected from the group consisting of immune deficiency disorders, hemophilia, thalassemia, sickle cell disease, blood disease, chronic granulomatous disorder, congenital blindness, lysosomal storage disease, muscular dystrophy, cancer, neurodegenerative disease, viral infections, bacterial infections, epidermolysis bullosa, heart disease, fat metabolism disorder, and diabetes, or a combination of these. In some embodiments, the disease is a cancer.

After obtaining the cell-free nucleic acid molecules, any of a number of different library preparation procedures for preparing nucleic acid molecules for sequencing may be performed on the cell-free nucleic acid molecules. Cell-free nucleic acid molecules may be processed before sequencing with one or more reagents (e.g., enzymes, adapters, tags (e.g. barcodes), probes, etc.). Tagged molecules may then be used in a downstream application, such as a sequencing reaction by which individual molecules may be tracked.

In some embodiments, the methods may further comprise an enrichment step prior to sequencing, whereby regions of the tagged molecules are selectively or non-selectively enriched. These sequences are referred to herein as "target sequences" or "targeted sequences." Target sequences may be known, partially known, or unknown prior to carrying-out the present methods. In some embodiments, the target sequence comprises a sequence that is mappable to (e.g., substantially aligns with) at least a portion of a reference sequence. In some cases, a target sequence may comprise regions from a subject's genome or transcriptome. In other cases, a target sequence may comprise regions from a therapeutic nucleic acid construct that comprise a sequence or sequences other than regions from a subject's genome or transcriptome. In some cases, a target sequence may comprise portions of genes or variants thereof, including, but not limited to *TP53, HBB, RPE65, and B-domain deleted factor VIII gene, DMD, UL123, APOB, SMN1, SMN2, ICAM-1, TLR9, IRS-1, VEGF, PDGFA, PDGFB, PDGFC, PDGFD,* and *TTR.* In some cases, a target sequence comprises a sequence of the construct designed to inhibit or block proteins encoded by a gene or a variant thereof, such as the genes previously described above. In some embodiments, the gene encoding for the chimeric antigen receptor or the sequence of the vector used in a CAR-T cell process may constitute the target sequence. Referring back to FIG. 1, an example of a target sequence is the TP53 cDNA or gene sequence within the vector.

Once sequencing data of the cell-free nucleic acid molecules is collected, one or more bioinformatics processes may be applied to the sequence data to detect the presence or absence of a therapeutic nucleic acid construct or fragments thereof. Such processes may include, but are not limited to, quantifying coverage amplification of a given sequence, determining over-representation or amplification of certain sequences among all sequence reads, identifying unmapped sequences in proximity to mapped sequences, detecting intron deletions within mapped reads, ascertaining the origin of unmapped reads, assembling high-confidence contigs of unmapped reads, and analyzing the size distribution of the sequence reads, or any combination of these methods. In some cases, sequence reads generated from a sequencing reaction can be aligned to a reference sequence for carrying out bioinformatics analysis. In some cases, reads can be aligned to the complete or partial sequence of the nucleic acid construct. In various aspects of bioinformatics analysis, one or more thresholds may be set to ensure quality. For example, an alignment threshold may be set such that only highly similar sequence reads (e.g., with 10 or less mismatches between a reference sequence and sequence reads) are mapped to a reference sequence. In some cases, sequence reads may be removed that cannot pass a quality threshold, e.g., based on chromatograms of sequence reads. In some cases, copy numbers or amounts of a given sequence may be quantified based on the number of sequence reads mapping or aligning to the given sequence. In some cases, over-representation of sequence(s) may be determined by comparing copy numbers or amounts of different sequences among all sequence reads. In some cases, based on the threshold set for alignment, unmapped sequence reads may be further analyzed. In some cases, a portion of the sequence read maps to the reference sequence. For those reads, the unmapped portion of the sequence read may be analyzed. In some cases, coordinates of mapped sequence reads on the reference genome may be determined. For example, position on a chromosome of a reference sequence may be determined. In some cases, a consensus sequence for unmapped sequence reads may be determined by assembling high-confidence contigs (e.g., by assembling sequence reads for both the stands of unmapped reads). In some cases, size distribution of unmapped sequence reads may be compared with size distribution of mapped sequence reads. In some cases, size distribution of unmapped sequence reads may be unimodal. In some cases, size distribution of mapped sequence reads may be bimodal. In certain embodiments, size distribution of sequence reads of cell-free nucleic acid molecules that are of therapeutic nucleic acid origin may be unimodal. In certain embodiments, size distribution of sequence reads of cell-free nucleic acid molecules that are of cellular origin may be bimodal. In some cases, more than one type of bioinformatics analysis may be used to detect the presence or absence of a therapeutic nucleic acid construct.

In certain embodiments, the nucleotide sequence of the therapeutic nucleic acid construct may be known, partially known, or unknown *a priori* to carrying out the methods described herein. In certain embodiments, the therapeutic nucleic acid construct may share part of its sequence with a reference sequence. The reference sequence may be a sequence of the human genome in some embodiments. An alternative or an additional reference sequence may be a sequence of a known segment of the therapeutic nucleic acid construct. In certain embodiments, the additional or alternative reference sequence may exclude a sequence that is shared (e.g., substantially aligns) with the human genome. Accordingly, in certain embodiments, depending on the sequences of the therapeutic nucleic acid construct that are known, partially known, or unknown prior to implementing the methods described herein, an appropriate reference sequence or sequences can be used for mapping sequence reads. In particular, a reference sequence may be selected that can distinguish the therapeutic nucleic acid construct over other nucleic acid sequences in the sample (i.e., differentiating reference sequence information). For example, for an expression vector, a portion of the sequence of the vector may be known, such as a promoter region, prior to administration of the vector to a patient. A reference sequence known to include the sequence of the promoter region of the vector can be used for mapping to the sequence reads from the promoter region to the reference sequence, thereby detecting the presence or absence of the vector.

In certain embodiments, sequencing reads are identified that do not align with a first reference sequence that originates from the human genome. These non-aligned sequence reads can then be mapped to a second reference sequence that originates from the therapeutic nucleic acid construct to detect the presence the therapeutic nucleic acid construct.

In certain embodiments, sequence reads may comprise sequences that map to (e.g., substantially align with) the differentiating portion of the second reference sequence originating from the therapeutic nucleic acid construct. These sequences can then be filtered out to improve the sensitivity of detecting sequences originating from the subject (e.g., fragments comprising targeted somatic mutations).

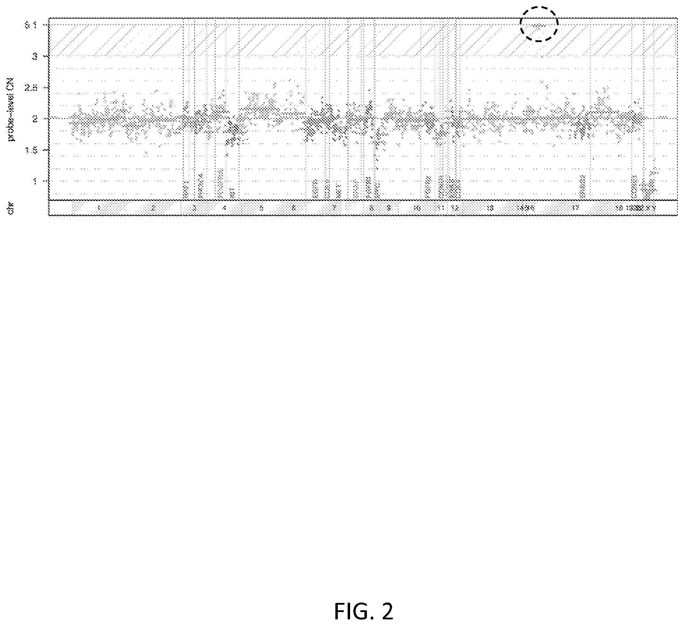

In certain embodiments, the number of sequence reads that align or map to the portion of the reference sequence shared by the nucleic acid construct may be quantified. Based on this quantification value, the increase in coverage of a sequence of interest may be determined by comparing the value to an expected threshold. Values above the threshold may represent an increase in coverage of a sequence. An example of this method is shown in FIG. 2 where an increase in coverage (>5) was measured for the TP53 gene on chromosome 17 (dashed circle) as compared to the baseline (^{~}2). Thus, the over representation value is indicative of a therapeutic nucleic acid construct being present in the sample.

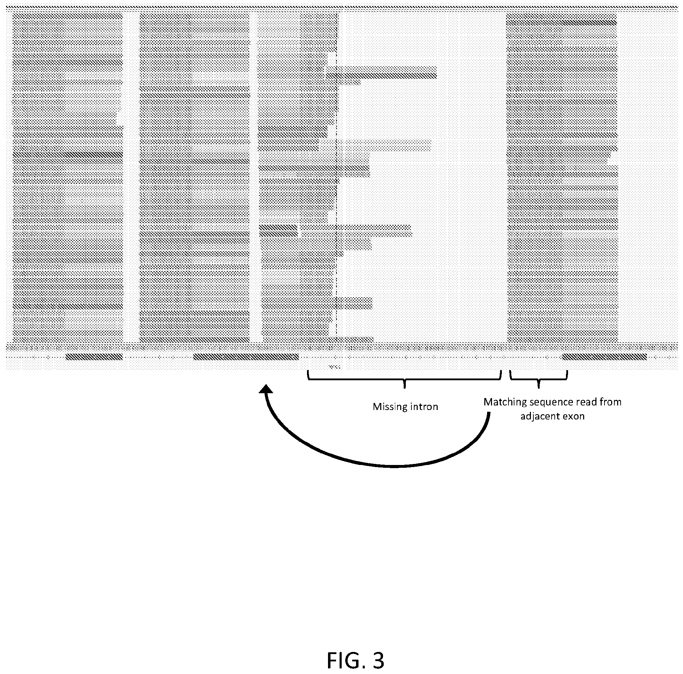

In other embodiments, only a portion of each sequence read may align or map to the reference sequence. In some cases, the sequence reads may align or map to only the exons of the reference sequence, but not to introns. This is typically the case with constructs comprising a sequence of an exon or cDNA sequence, such as the TP53 gene sequence of FIG. 1. An example of detecting intron deletion is provided in FIG. 3, where the sequence reads are mapped to the reference sequence. As can be seen, a portion of the sequence reads do not align to an intron sequence of the reference sequence. Instead, the exon sequence adjacent to one side of the deleted intron is part of the exon sequence that is adjacent to the other side of the deleted intron. Therefore, detecting intron deletions may be indicative of a therapeutic nucleic acid construct being present in the sample.

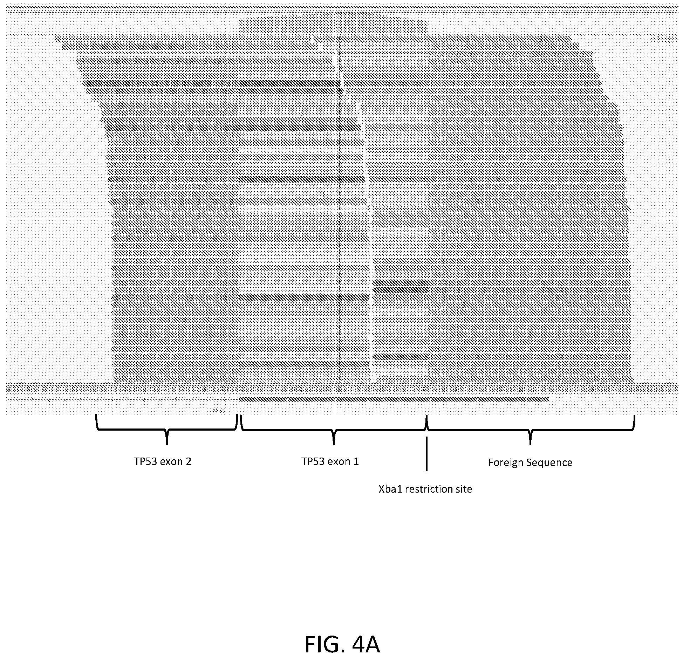

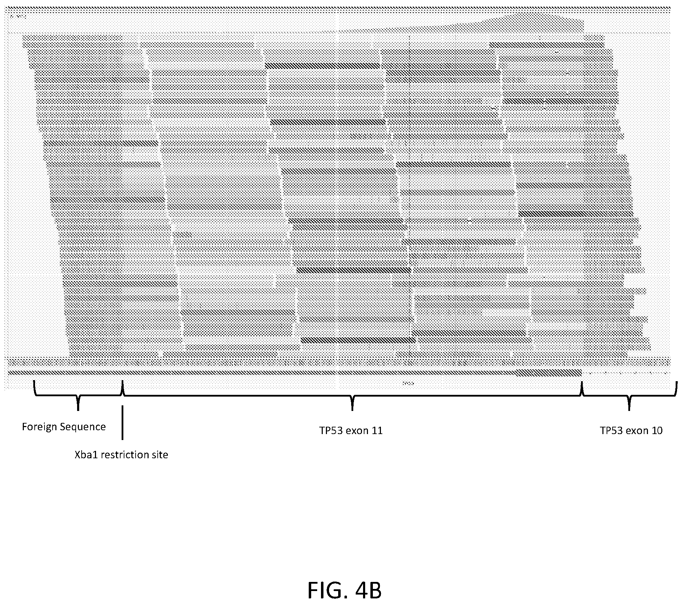

In other cases where only a portion of each sequence read may align or map to the reference sequence is where the sequence read overlaps a junction (breakpoint) between a mapped sequence and an unmapped sequence. Common junctions may be found, for example, in the untranslated region (UTR) of a gene or at locations targeted by common restriction enzymes. FIG. 4A and FIG. 4B show mapping diagrams where a foreign (unmapped) sequence adjacent to an enzyme restriction site (Xba1) was found in proximity to mapped sequence reads TP53 exon 1 (FIG. 4A) and TP53 exon 11 (FIG. 4B). Accordingly, the location and proximity of an unmapped sequence read to a mapped sequence read may be used to determine the presence of the therapeutic nucleic acid construct.

In certain embodiments, some of the sequence reads may not align or map to the reference sequence. Unmapped reads may be further investigated to determine their origin. Such methods may include, for example, performing a BLAST search. Results from the search may indicate that the unmapped reads relate to a sequence used in the therapeutic nucleic acid construct. Unmapped reads may be non-genomic sequences. For example, it may be determined that unmapped sequence reads relate to an origin of replication sequence (e.g., SV40 ori) that is used in therapeutic nucleic acid constructs. Additional methods for using unmapped sequence reads may include, for example, forming an assembly of contiguous unmapped reads. An assembly of a certain number of unmapped reads may be of sufficient quality/high confidence to indicate the presence of a therapeutic nucleic acid construct.

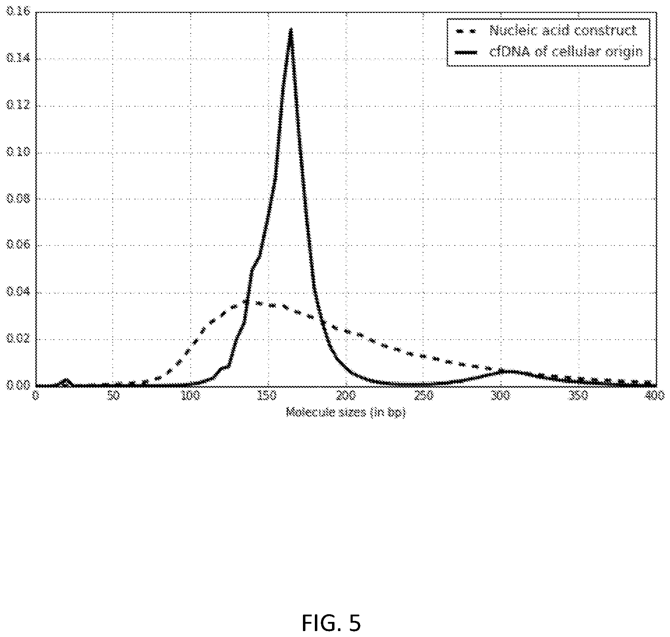

In further embodiments, the size distribution of the sequence reads may be determined and compared to expected size distributions of cell-free nucleic acids. Deviations from the expected size distribution may be indicative of the presence of the therapeutic nucleic acid construct. An example of such a deviation is shown in FIG. 5, where a TP53 vector is detected by comparing the different size distributions of the sequence reads of cfDNA of cellular origin (solid line) against the TP53 plasmid sequence reads (dotted line) found as part of the cell-free nucleic acid molecules in the biological sample. This analysis may be performed with or without mapping.

A composition for administering to a subject can comprise: a polynucleotide having known sequence (e.g., a control nucleic acid or the like) and a composition comprising a therapeutic nucleic acid construct. The known sequence can be a synthetic sequence. The known sequence can be a sequence that does not align to a reference sequence for a subject. The known sequence can be a sequence that does not align to a therapeutic nucleic acid construct.

A polynucleotide having a known sequence (e.g., a control nucleic acid or the like) can be used to assess the quality of the therapeutic composition. For example, the polynucleotide having the known sequence at a specific concentration can be added in an amount proportional to the therapeutic nucleic acid construct in the therapeutic composition. The polynucleotide having the known sequence can be sequenced and the amounts of the polynucleotide having the known sequence can be used to determine, e.g., as a function of time, the stability of the composition under different conditions, such as storage conditions, reagents, sample buffer, etc. In another example, the amounts of the polynucleotide having the known sequence in different preparations can be determined to assess lot-to-lot variations and thus, differences in the concentration of the therapeutic nucleic acid constructs between the preparations can be inferred.

A polynucleotide having a known sequence can be used for accurately determining the concentration of the therapeutic nucleic acid construct when the composition is administered to a subject. For example, the polynucleotide having a known sequence at a specific concentration can be added in an amount proportional to the therapeutic nucleic acid construct in the therapeutic composition. Since, the amounts of the polynucleotide having the known sequence is known, this can be used for determining the amounts of the
therapeutic nucleic acid construct in the subject without sequencing the therapeutic nucleic acid construct. In another example, the amounts of the polynucleotide having the known sequence can be used for normalizing the amounts of the therapeutic nucleic acid construct. This is useful when comparing amounts of the therapeutic nucleic acid construct at different time points or among different individuals. This can be useful when assessing efficacy of treatments. For example, the amounts of different therapeutic nucleic acid constructs among different cohorts can be accurately determined using the known sequence. Efficacy of the treatments can then be determined by comparing amounts of the polynucleotide having the known sequence and/or the therapeutic nucleic acid constructs among the cohorts undergoing different treatment regimens.

In some embodiments, the known sequence can be useful in determining the cause of the absence of sequencing reads from the therapeutic nucleic acid construct or cell-free nucleic acid molecules. For example, the presence of sequence reads from the known sequence can confirm the functioning of the sequencer. On the contrary, absence of sequence reads from the known sequence can indicate sequencing error.

In some embodiments, a polynucleotide having a known sequence can be at least about 1 nucleotide (nt) to about 1000 nt. In some cases, a polynucleotide having a known sequence can be about 10 nt to about 800 nt. In some cases, a polynucleotide having a known sequence can be about 20 nt to about 600 nt. In some cases, a polynucleotide having a known sequence can be about 30 nt to about 400 nt. In some cases, a polynucleotide having a known sequence can be about 40 nt to about 300 nt. In some cases, a polynucleotide having a known sequence can be about 50 nt to about 200 nt nucleotides. In some cases, a polynucleotide having a known sequence can be about 60 nt to about 100 nt. In some cases, a polynucleotide having a known sequence is about the length of average length of the cell-free nucleic acid. For example, the polynucleotide having a known sequence can be about 200 nt.

The disclosure further provides that the method steps disclosed herein are optionally adapted for performance using systems and/or computer readable media disclosed herein. In certain aspects, a system may comprise a controller comprising, or capable of accessing, computer readable media comprising non-transitory computer-executable instructions, which, when executed by at least one electronic processor perform at least one of the methods described herein.

In some embodiments, sequence reads are generated by a sequencer, such as a DNA sequencer. In some embodiments, the sequencer is designed to perform high-throughput sequencing, such as next generation sequencing. In some embodiments, the system comprises adapter tagged cell-free nucleic acid molecules in the sequencers. In some embodiments, the adapter tagged cell-free nucleic acid molecules are sourced from one subject or a plurality of subjects. In some embodiments, the cell-free nucleic acid molecules from the sample bear unique or non-unique barcodes.

In some embodiments, the methods and systems described herein utilize a digital processing device. In further embodiments, the digital processing device includes one or more hardware central processing units (CPUs) or general purpose graphics processing units (GPGPUs) that carry out the device's functions. In still further embodiments, the digital processing device further comprises an operating system configured to perform executable instructions. In some embodiments, the digital processing device is optionally connected to a computer network. In further embodiments, the digital processing device is optionally connected to the Internet such that it accesses the World Wide Web. In still further embodiments, the digital processing device is optionally connected to a cloud computing infrastructure. In other embodiments, the digital processing device is optionally connected to an intranet. In other embodiments, the digital processing device is optionally connected to a data storage device. In accordance with the description herein, suitable digital processing devices include, by way of non-limiting examples, server computers, desktop computers, laptop computers, notebook computers, handheld computers, Internet appliances, mobile smartphones, and tablet computers.

In some embodiments, the digital processing device includes an operating system configured to perform executable instructions. The operating system is, for example, software, including programs and data, which manages the device's hardware and provides services for execution of applications. Those of skill in the art will recognize that suitable server operating systems include, by way of non-limiting examples, FreeBSD, OpenBSD, NetBSD^{®}, Linux, Apple^{®} Mac OS X Server^{®}, Oracle^{®} Solaris^{®}, Windows Server^{®}, and Novell^{®} NetWare^{®}. Those of skill in the art will recognize that suitable personal computer operating systems include, by way of non-limiting examples, Microsoft^{®} Windows^{®}, Apple^{®} Mac OS X^{®}, UNIX^{®}, and UNIX-like operating systems such as GNU/Linux^{®}. In some embodiments, the operating system is provided by distributed computing, such as cloud computing. Those of skill in the art will also recognize that suitable mobile smart phone operating systems include, by way of non-limiting examples, Nokia^{®} Symbian^{®} OS, Apple^{®} iOS^{®}, Research In Motion^{®} BlackBerry OS^{®}, Google^{®} Android^{®}, Microsoft^{®} Windows Phone^{®} OS, Microsoft^{®} Windows Mobile^{®} OS, Linux^{®}, and Palm^{®} WebOS^{®}.

In some embodiments, the device includes a storage and/or memory device. The storage and/or memory device is one or more physical apparatuses used to store data or programs on a temporary or permanent basis. In some embodiments, the device is volatile memory and requires power to maintain stored information. In some embodiments, the device is non-volatile memory and retains stored information when the digital processing device is not powered. In further embodiments, the non-volatile memory comprises flash memory. In some embodiments, the non-volatile memory comprises dynamic random-access memory (DRAM). In some embodiments, the non-volatile memory comprises ferroelectric random access memory (FRAM). In some embodiments, the non-volatile memory comprises phase-change random access memory (PRAM). In other embodiments, the device is a storage device including, by way of non-limiting examples, CD-ROMs, DVDs, flash memory devices, magnetic disk drives, magnetic tapes drives, optical disk drives, and cloud computing based storage. In further embodiments, the storage and/or memory device is a combination of devices such as those disclosed herein.

In some embodiments, the digital processing device includes an electronic display to send visual information to a user. In some embodiments, the display is a liquid crystal display (LCD). In further embodiments, the display is a thin film transistor liquid crystal display (TFT-LCD). In some embodiments, the display is an organic light emitting diode (OLED) display. In various further embodiments, on OLED display is a passive-matrix OLED (PMOLED) or active-matrix OLED (AMOLED) display. In some embodiments, the display is a plasma display. In other embodiments, the display is a video projector. In yet other embodiments, the display is a head-mounted display in communication with the digital processing device, such as a VR headset. In further embodiments, suitable VR headsets include, by way of non-limiting examples, HTC Vive, Oculus Rift, Samsung Gear VR, Microsoft HoloLens, Razer OSVR, FOVE VR, Zeiss VR One, Avegant Glyph, Freefly VR headset, and the like. In still further embodiments, the display is a combination of devices such as those disclosed herein.

In some embodiments, the digital processing device includes an input device to receive information from a user. In some embodiments, the input device is a keyboard. In some embodiments, the input device is a pointing device including, by way of non-limiting examples, a mouse, trackball, track pad, joystick, game controller, or stylus. In some embodiments, the input device is a touch screen or a multi-touch screen. In other embodiments, the input device is a microphone to capture voice or other sound input. In other embodiments, the input device is a video camera or other sensor to capture motion or visual input. In further embodiments, the input device is a Kinect, Leap Motion, or the like. In still further embodiments, the input device is a combination of devices such as those disclosed herein.

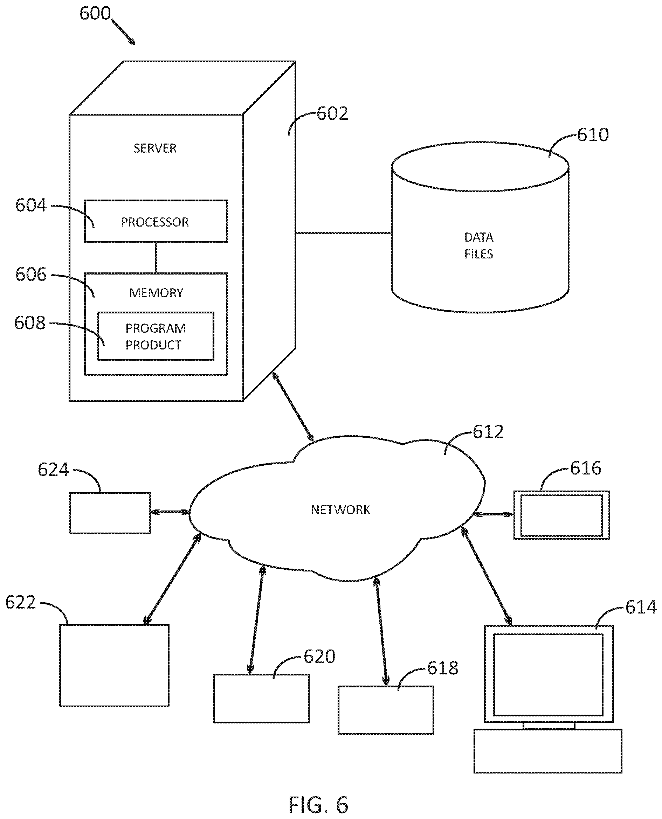

To further illustrate, FIG. 6 provides a schematic diagram of an exemplary system suitable for use with implementing at least aspects of the methods disclosed in this application. As shown, system 600 includes at least one controller or computer, e.g., server 602 (e.g., a search engine server), which includes processor 604 and memory, storage device, or memory component 606, and one or more other communication devices 614 and 616 (e.g., client-side computer terminals, telephones, tablets, laptops, other mobile devices, etc.) positioned remote from and in communication with the remote server 602, through electronic communication network 612, such as the internet or other internetwork. Communication devices 614 and 616 typically include an electronic display (e.g., an internet enabled computer or the like) in communication with, e.g., server 602 computer over network 612 in which the electronic display comprises a user interface (e.g., a graphical user interface (GUI), a web-based user interface, and/or the like) for displaying results upon implementing the methods described herein. In certain embodiments, communication networks also encompass the physical transfer of data from one location to another, for example, using a hard drive, thumb drive, or other data storage mechanism. System 600 also includes program product 608 stored on a computer or machine readable medium, such as, for example, one or more of various types of memory, such as memory 606 of server 602, that is readable by the server 602, to facilitate, for example, a guided search application or other executable by one or more other communication devices, such as 614 (schematically shown as a desktop or personal computer) and 616 (schematically shown as a tablet computer). In some embodiments, system 600 optionally also includes at least one database server, such as, for example, server 610 associated with an online website having data stored thereon (e.g., reference sequence information, etc.) searchable either directly or through search engine server 602. System 600 optionally also includes one or more other servers positioned remotely from server 602, each of which are optionally associated with one or more database servers 610 located remotely or located local to each of the other servers. The other servers can beneficially provide service to geographically remote users and enhance geographically distributed operations.

As understood by those of ordinary skill in the art, memory 606 of the server 602 optionally includes volatile and/or nonvolatile memory including, for example, RAM, ROM, and magnetic or optical disks, among others. It is also understood by those of ordinary skill in the art that although illustrated as a single server, the illustrated configuration of server 602 is given only by way of example and that other types of servers or computers configured according to various other methodologies or architectures can also be used. Server 602 shown schematically in FIG. 6, represents a server or server cluster or server farm and is not limited to any individual physical server. The server site may be deployed as a server farm or server cluster managed by a server hosting provider. The number of servers and their architecture and configuration may be increased based on usage, demand and capacity requirements for the system 600. As also understood by those of ordinary skill in the art, other user communication devices 614 and 616 in these embodiments, for example, can be a laptop, desktop, tablet, personal digital assistant (PDA), cell phone, server, or other types of computers. As known and understood by those of ordinary skill in the art, network 612 can include an internet, intranet, a telecommunication network, an extranet, or world wide web of a plurality of computers/servers in communication with one or more other computers through a communication network, and/or portions of a local or other area network.

As further understood by those of ordinary skill in the art, exemplary program product or machine readable medium 608 is optionally in the form of microcode, programs, cloud computing format, routines, and/or symbolic languages that provide one or more sets of ordered operations that control the functioning of the hardware and direct its operation. Program product 608, according to an exemplary embodiment, also need not reside in its entirety in volatile memory, but can be selectively loaded, as necessary, according to various methodologies as known and understood by those of ordinary skill in the art.

As further understood by those of ordinary skill in the art, the term "computer-readable medium" or "machine-readable medium" refers to any medium that participates in providing instructions to a processor for execution. To illustrate, the term "computer-readable medium" or "machine-readable medium" encompasses distribution media, cloud computing formats, intermediate storage media, execution memory of a computer, and any other medium or device capable of storing program product 608 implementing the functionality or processes of various embodiments of the present disclosure, for example, for reading by a computer. A "computer-readable medium" or "machine-readable medium" may take many forms, including but not limited to, non-volatile media, volatile media, and transmission media. Non-volatile media includes, for example, optical or magnetic disks. Volatile media includes dynamic memory, such as the main memory of a given system. Transmission media includes coaxial cables, copper wire and fiber optics, including the wires that comprise a bus. Transmission media can also take the form of acoustic or light waves, such as those generated during radio wave and infrared data communications, among others. Exemplary forms of computer-readable media include a floppy disk, a flexible disk, hard disk, magnetic tape, a flash drive, or any other magnetic medium, a CD-ROM, any other optical medium, punch cards, paper tape, any other physical medium with patterns of holes, a RAM, a PROM, and EPROM, a FLASH-EPROM, any other memory chip or cartridge, a carrier wave, or any other medium from which a computer can read.

Program product 608 is optionally copied from the computer-readable medium to a hard disk or a similar intermediate storage medium. When program product 608, or portions thereof, are to be run, it is optionally loaded from their distribution medium, their intermediate storage medium, or the like into the execution memory of one or more computers, configuring the computer(s) to act in accordance with the functionality or method of various embodiments. All such operations are well known to those of ordinary skill in the art of, for example, computer systems.

To further illustrate, in certain embodiments, this application provides systems that include one or more processors, and one or more memory components in communication with the processor. The memory component typically includes one or more instructions that, when executed, cause the processor to provide information that causes test sequence information, reference sequence information, and/or the like to be displayed (e.g., via communication devices 614, 616, or the like) and/or receive information from other system components and/or from a system user (e.g., via communication devices 614, 616, or the like).

In some embodiments, program product 608 includes non-transitory computer-executable instructions which, when executed by electronic processor 604 perform at least: (i) receiving, over the communication network, the sequencing reads generated by the nucleic acid sequencer, (ii) aligning said sequencing reads to a reference sequence from a human genome, and (iii) detecting the presence of the nucleic acid construct based on the alignment of sequencing reads to the reference sequence except in the region of an intron. Additional computer readable media embodiments are described herein.

System 600 also typically includes additional system components that are configured to perform various aspects of the methods described herein. In some of these embodiments, one or more of these additional system components are positioned remote from and in communication with the remote server 602 through electronic communication network 612, whereas in other embodiments, one or more of these additional system components are positioned local, and in communication with server 602 (i.e., in the absence of electronic communication network 612) or directly with, for example, desktop computer 614.

In some embodiments, for example, additional system components include sample preparation component 618 is operably connected (directly or indirectly (e.g., via electronic communication network 612)) to controller 602. Sample preparation component 618 is configured to prepare the nucleic acids in samples (e.g., prepare libraries of nucleic acids) to be amplified and/or sequenced by a nucleic acid amplification component (e.g., a thermal cycler, etc.) and/or a nucleic acid sequencer. In certain of these embodiments, sample preparation component 618 is configured to isolate nucleic acids from other components in a sample, to attach one or more adapters comprising barcodes to nucleic acids as described herein, selectively enrich one or more regions from a genome or transcriptome prior to sequencing, and/or the like.

In certain embodiments, system 600 also includes nucleic acid amplification component 620 (e.g., a thermal cycler, etc.) operably connected (directly or indirectly (e.g., via electronic communication network 612)) to controller 602. Nucleic acid amplification component 620 is configured to amplify nucleic acids in samples from subjects. For example, nucleic acid amplification component 620 is optionally configured to amplify selectively enriched regions from a genome or transcriptome in the samples as described herein.

System 600 also typically includes at least one nucleic acid sequencer 622 operably connected (directly or indirectly (e.g., via electronic communication network 612)) to controller 602. Nucleic acid sequencer 622 is configured to provide the sequence information from nucleic acids (e.g., amplified nucleic acids) in samples from subjects. Essentially any type of nucleic acid sequencer can be adapted for use in these systems. For example, nucleic acid sequencer 622 is optionally configured to perform pyrosequencing, single-molecule sequencing, nanopore sequencing, semiconductor sequencing, sequencing-by-ligation, sequencing-by-hybridization, or other techniques on the nucleic acids to generate sequencing reads. Optionally, nucleic acid sequencer 622 is configured to group sequence reads into families of sequence reads, each family comprising sequence reads generated from a nucleic acid in a given sample. In some embodiments, nucleic acid sequencer 622 uses a clonal single molecule array derived from the sequencing library to generate the sequencing reads. In certain embodiments, nucleic acid sequencer 622 includes at least one chip having an array of microwells for sequencing a sequencing library to generate sequencing reads.

To facilitate complete or partial system automation, system 600 typically also includes material transfer component 624 operably connected (directly or indirectly (e.g., via electronic communication network 612)) to controller 602. Material transfer component 624 is configured to transfer one or more materials (e.g., nucleic acid samples, amplicons, reagents, and/or the like) to and/or from nucleic acid sequencer 622, sample preparation component 618, and nucleic acid amplification component 620.

Additional details relating to computer systems and networks, databases, and computer program products are also provided in, for example, Peterson, Computer Networks: A Systems Approach, Morgan Kaufmann, 5th Ed. (2011), Kurose, Computer Networking: A Top-Down Approach, Pearson, 7th Ed. (2016), Elmasri, Fundamentals of Database Systems, Addison Wesley, 6th Ed. (2010), Coronel, Database Systems: Design, Implementation, & Management, Cengage Learning, 11th Ed. (2014), Tucker, Programming Languages, McGraw-Hill Science/Engineering/Math, 2nd Ed. (2006), and Rhoton, Cloud Computing Architected: Solution Design Handbook, Recursive Press (2011).

### II. General Features of the Methods

### A. Samples

A sample can be any biological sample isolated from a subject. Samples can include body tissues, such as known or suspected solid tumors, whole blood, platelets, serum, plasma, stool, red blood cells, white blood cells or leucocytes, endothelial cells, tissue biopsies, cerebrospinal fluid synovial fluid, lymphatic fluid, ascites fluid, interstitial or extracellular fluid, the fluid in spaces between cells, including gingival crevicular fluid, bone marrow, pleural effusions, cerebrospinal fluid, saliva, mucous, sputum, semen, sweat, urine. Samples are preferably body fluids, particularly blood and fractions thereof, and urine. Samples may also include nucleic acids shed from tumors, e.g., circulating tumor DNA (ctDNA). The nucleic acids can include DNA and RNA and can be in double- and single-stranded forms. The sample may also include a therapeutic nucleic acid construct. A sample can be in the form originally isolated from a subject or can have been subjected to further processing to remove or add components, such as cells, enrich for one component relative to another, or convert one form of nucleic acid to another, such as RNA to DNA or single-stranded nucleic acids to double-stranded. Thus, for example, a body fluid for analysis is plasma or serum containing cell-free nucleic acids, e.g., cell-free DNA (cfDNA).

The volume of plasma can depend on the desired read depth for sequenced regions. Exemplary volumes are 0.4-40 ml, 5-20 ml, 10-20 ml. For example, the volume can be 0.5 ml, 1 mL, 5 ml, 10 ml, 20 ml, 30 ml, or 40 ml. A volume of sampled plasma may be 5 to 20 ml.

The sample can comprise various amounts of nucleic acid that contains genome equivalents. For example, a sample of about 30 ng DNA can contain about 10,000 (10⁴) haploid human genome equivalents and, in the case of cfDNA, about 200 billion (2x10¹¹) individual polynucleotide molecules. Similarly, a sample of about 100 ng of DNA can contain about 30,000 haploid human genome equivalents and, in the case of cfDNA, about 600 billion individual molecules.

A sample can comprise nucleic acids from different sources, e.g., from cells or from a foreign object. A sample can comprise nucleic acids carrying mutations. For example, a sample can comprise DNA carrying germline mutations and/or somatic mutations. A sample can comprise DNA carrying cancer-associated mutations (e.g., cancer-associated somatic mutations).

Exemplary amounts of cell-free nucleic acids in a sample before amplification range from about 1 fg to about 1 ug, e.g., 1 pg to 200 ng, 1 ng to 100 ng, 10 ng to 1000 ng. For example, the amount can be up to about 600 ng, up to about 500 ng, up to about 400 ng, up to about 300 ng, up to about 200 ng, up to about 100 ng, up to about 50 ng, or up to about 20 ng of cell-free nucleic acid molecules. The amount can be at least 1 fg, at least 10 fg, at least 100 fg, at least 1 pg, at least 10 pg, at least 100 pg, at least 1 ng, at least 10 ng, at least 100 ng, at least 150 ng, or at least 200 ng of cell-free nucleic acid molecules. The amount can be up to 1 femtogram (fg), 10 fg, 100 fg, 1 picogram (pg), 10 pg, 100 pg, 1 ng, 10 ng, 100 ng, 150 ng, or 200 ng of cell-free nucleic acid molecules. The method can comprise obtaining 1 femtogram (fg) to 200 ng.

In certain embodiments, the amount of cell-free nucleic acids in the sample is between about 5 ng and 300 ng.

Cell-free nucleic acids of cellular origin have an exemplary size distribution of about 100-500 nucleotides, with molecules of 110 to about 230 nucleotides representing about 90% of molecules, with a mode of about 168 nucleotides and a second minor peak in a range between 240 to 440 nucleotides. Cell-free nucleic acids of cellular origin can be about 160 to about 180 nucleotides, or about 320 to about 360 nucleotides, or about 440 to about 480 nucleotides.

Cell-free nucleic acids can be isolated from bodily fluids through a partitioning step in which cell-free nucleic acids, as found in solution, are separated from intact cells and other non-soluble components of the bodily fluid. Partitioning may include techniques such as centrifugation or filtration. Alternatively, cells in bodily fluids can be lysed and cell-free and cellular nucleic acids processed together. Generally, after addition of buffers and wash steps, cell-free nucleic acids can be precipitated with an alcohol. Further clean up steps may be used such as silica based columns to remove contaminants or salts. Non-specific bulk carrier nucleic acids, for example, may be added throughout the reaction to optimize certain aspects of the procedure such as yield. After such processing, samples can include various forms of nucleic acid including double-stranded DNA, single stranded DNA and single stranded RNA. Optionally, single stranded DNA and RNA can be converted to double-stranded forms so they are included in subsequent processing and analysis steps.

### B. Tags

Tags providing molecular identifiers (also referred herein as "molecular barcodes") can be incorporated into or otherwise joined to adapters by chemical synthesis, ligation, overlap extension PCR among other methods. Generally, assignment of unique or non- molecular barcodes in reactions follows methods and systems described by US patent applications 20010053519, 20110160078, and U.S. Pat. No. 6,582,908 and U.S. Pat. No. 7,537,898 and US 9,598,731.

Tags can be linked to sample nucleic acids randomly or non-randomly. In some cases, they are introduced at an expected ratio of molecular barcodes (e.g., a combination of molecular barcodes). The collection of molecular barcodes can be unique, e.g., all the barcodes have the same nucleotide sequence. The collection of molecular barcodes can be non-unique, e.g., some of the barcodes have the same nucleotide sequence, and some of the barcodes have different nucleotide sequence. For example, the barcodes may be loaded so that more than 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 barcodes are loaded per genome sample. In some cases, the barcodes may be loaded so that less than 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 barcodes are loaded per genome sample. In some cases, the average number of barcodes loaded per sample genome is less than, or greater than, about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 barcodes per genome sample.

A preferred format uses 20-50 different tags, ligated to both ends of a target molecule creating 20-50 x 20-50 tags. Such numbers of tags are sufficient that different molecules having the same start and stop points have a high probability (e.g., at least 94%, 99.5%, 99.99%, 99.999%) of receiving different combinations of tags.

In some cases, molecular barcodes may be predetermined or random or semi-random sequence oligonucleotides. In other cases, a plurality of barcodes may be used such that barcodes are not necessarily unique to one another in the plurality. In this example, barcodes may be attached (e.g., by ligation or PCR amplification) to individual molecules such that the combination of the barcodes and the sequence it may be attached to creates a unique sequence that may be individually tracked. As described herein, detection of non-uniquely tagged barcodes in combination with sequence data of beginning (start) and end (stop) portions of sequence reads of the cell-free nucleic acid molecule, or genomic coordinates as mapped to a reference sequence, may allow assignment of a unique identity to a particular molecule. The length, or number of base pairs, of an individual sequence read may also be used to assign a unique identity to such a molecule. As described herein, fragments from a single strand of nucleic acid having been assigned a unique identity, may thereby permit subsequent identification of fragments from the parent strand, and/or a complementary strand.

One or more amplifications can be applied to introduce tags, such as molecular barcodes and/or sample indexes, to a nucleic acid molecule using conventional nucleic acid amplification methods. The amplification can be conducted in one or more reaction mixtures. Molecular barcodes and sample indexes can be introduced simultaneously, or in any sequential order. Molecular barcodes and sample indexes can be introduced prior to and/or after sequence capturing (e.g. enrichment). In some embodiments, only the molecular barcodes are introduced prior to probe capturing while the sample indexes are introduced after sequence capturing. In some cases, both the molecular barcodes and the sample indexes are introduced prior to probe capturing. In some cases, the sample indexes are introduced after sequence capturing. Usually, sequence capturing involves introducing a single-stranded nucleic acid molecule complementary to a targeted sequence. Typically, the amplifications generate a plurality of non-uniquely or uniquely tagged nucleic acid amplicons with molecular barcodes and sample indexes at a size ranging from 200 nt to 700 nt, 250 nt to 350 nt, or 320 nt to 550 nt. In some embodiments, the amplicons have a size of about 300 nt. In some embodiments, the amplicons have a size of about 500 nt.

### C. Amplification

Sample nucleic acids flanked by adapters can be amplified by PCR and other amplification methods typically primed from primers binding to primer binding sites in adapters flanking a nucleic acid molecule to be amplified. Amplification methods can involve cycles of extension, denaturation and annealing resulting from thermocycling or can be isothermal as in transcription mediated amplification. Other amplification methods include the ligase chain reaction, strand displacement amplification, nucleic acid sequence based amplification, and self-sustained sequence based replication.

### D. Enrichment

Sequences can be enriched prior to sequencing. Enrichment can be performed for specific target regions or nonspecifically (i.e., target sequences). In some embodiments, targeted regions of interest may be enriched with capture probes ("baits") selected for one or more bait set panels using a differential tiling and capture scheme. A differential tiling and capture scheme uses bait sets of different relative concentrations to differentially tile (e.g., at different "resolutions") across genomic regions associated with baits, subject to a set of constraints (e.g., sequencer constraints such as sequencing load, utility of each bait, etc.), and capture them at a desired level for downstream sequencing. These targeted genomic regions of interest may include natural or synthetic nucleotide sequences of the nucleic acid construct. In some embodiments, biotin-labeled beads with probes to one or more regions of interest can be used to capture target sequences, optionally followed by amplification of those regions, to enrich for the regions of interest.

Sequence capture typically involves the use of oligonucleotide probes that hybridize to the target sequence. A probe set strategy can involve tiling the probes across a region of interest. Such probes can be, e.g., about 60 to 120 bases long. The set can have a depth of about 2x, 3x, 4x, 5x, 6x, 8x, 9x, 10x, 15x, 20x, 50x or more. The effectiveness of sequence capture depends, in part, on the length of the sequence in the target molecule that is complementary (or nearly complementary) to the sequence of the probe.

### E. Sequencing

Sample nucleic acids flanked by adapters with or without prior amplification can be subject to sequencing. Sequencing methods include, for example, Sanger sequencing, high-throughput sequencing, pyrosequencing, sequencing-by-synthesis, single-molecule sequencing, nanopore sequencing, semiconductor sequencing, sequencing-by-ligation, sequencing-by-hybridization, RNA-Seq (Illumina), Digital Gene Expression (Helicos), Next generation sequencing, Single Molecule Sequencing by Synthesis (SMSS) (Helicos), massively-parallel sequencing, Clonal Single Molecule Array (Solexa), shotgun sequencing, Ion Torrent, Oxford Nanopore, Roche Genia, Maxim-Gilbert sequencing, primer walking, sequencing using PacBio, SOLiD, Ion Torrent, or Nanopore platforms. Sequencing reactions can be performed in a variety of sample processing units, which may be multiple lanes, multiple channels, multiple wells, or other mean of processing multiple sample sets substantially simultaneously. Sample processing unit can also include multiple sample chambers to enable processing of multiple runs simultaneously.

The sequencing reactions can be performed on one or more fragments types known to contain markers of cancer of other disease. The sequencing reactions can also be performed on any nucleic acid fragments present in the sample. The sequence reactions may provide for sequence coverage of the genome of at least 5%, 10%, 15%, 20%, 25%, 30%, 40%, 50%, 60%, 70%, 80%, 90%, 95%, 99%, 99.9% or 100%. In other cases, sequence coverage of the genome may be less than 5%, 10%, 15%, 20%, 25%, 30%, 40%, 50%, 60%, 70%, 80%, 90%, 95%, 99%, 99.9% or 100%.

Simultaneous sequencing reactions may be performed using multiplex sequencing. In some cases, cell free polynucleotides may be sequenced with at least 1000, 2000, 3000, 4000, 5000, 6000, 7000, 8000, 9000, 10000, 50000, 100,000 sequencing reactions. In other cases, cell free polynucleotides may be sequenced with less than 1000, 2000, 3000, 4000, 5000, 6000, 7000, 8000, 9000, 10000, 50000, 100,000 sequencing reactions. Sequencing reactions may be performed sequentially or simultaneously. Subsequent data analysis may be performed on all or part of the sequencing reactions. In some cases, data analysis may be performed on at least 1000, 2000, 3000, 4000, 5000, 6000, 7000, 8000, 9000, 10000, 50000, 100,000 sequencing reactions. In other cases, data analysis may be performed on less than 1000, 2000, 3000, 4000, 5000, 6000, 7000, 8000, 9000, 10000, 50000, 100,000 sequencing reactions. An exemplary read depth is 1000-50000 reads per locus (base).

### F. Analysis

In some embodiments, raw sequencing data may comprise sets of sequence reads, which can be provided in various file formats, such as FASTQ, VCF, CRAM or BAM. Files with the raw sequencing data may include sequence data for one strand or both strands, such as in paired-end reads. In one example, the raw sequencing data is provided in a FASTQ file for both strands, i.e., sense and antisense strands generated from paired-end sequencing procedure. The files may include additional symbols providing information about the quality of reads and may also provide a quality score. The raw sequencing data of each polynucleotide molecule may be saved on a local drive, in cloud or a server.

In some cases, sequence reads generated from a sequencing reaction can be aligned or mapped to a reference sequence for carrying out bioinformatics analysis. The reference sequence is often a known sequence, e.g., a known whole or partial genome sequence from an object, whole genome sequence of a human subject. The reference sequence can be hG19. The sequenced nucleic acids can represent sequences determined directly for a nucleic acid in a sample, or a consensus of sequences of amplification products of such a nucleic acid, as described above. A comparison can be performed at one or more designated positions on a reference sequence.

Sequence reads may be aligned to a reference sequence using mapping tools, non-limiting examples of which may include Burrow's Wheeler Transform (BWA), Novoalign, Bowtie. The mapping tools generate an alignment file describing alignment parameters used, position of the sequence reads (such as coordinates) on to the reference sequence and a quality score of mapping. The alignment parameters, such as number of differences allowed between the sequencing read and the reference sequence, number of gaps allowed and gap opening penalty, number of gap extensions, and the like, may be defined by a user. In one instance, BWA mapping tool with default alignment parameters is used to align the reads to a human reference genome, such as hg19. BWA tool provides an output file, a BAM file that includes alignment statistics. Alignment statistics may include coordinates of the reference sequence to which the processed reads align to. Alignment statistics may also provide a MapQ score to inform uniqueness of the reads when mapped to the reference sequence. The processed reads may then be sorted using the molecular barcodes and the coordinates on the reference sequence.

A subset of sequenced nucleic acids can be identified including a position corresponding with a designated position of the reference sequence when the respective sequences are maximally aligned. Within such a subset it can be determined which, if any, sequenced nucleic acids include a nucleotide variation at the designated position, and optionally which if any, include a reference nucleotide (i.e., same as in the reference sequence). The comparison can be repeated for any designated position of interest in the reference sequence. Sometimes a comparison can be performed for designated positions occupying at least 20, 100, 200, or 300 contiguous positions on a reference sequence, e.g., 20-500, or 50-300 contiguous positions.

A sample may be contacted with a sufficient number of different molecular barcodes that there is a low probability (e.g., < 1 or 0.1 %) that any two copies of the same nucleic acid receive the same combination of an adapter containing a molecular barcode from the adapters linked at one end or both ends. The use of adapters in this manner may permit grouping of sequence reads with the same start and stop points that are aligned (or mapped) to a reference sequence and linked to the same combination of molecular barcodes into families of reads generated from the same original molecule. Such a family may represent sequences of amplification products of a nucleic acid in the sample before amplification.

Sequences of family members can be compiled to derive consensus nucleotide(s) or a complete consensus sequence for a nucleic acid molecule in the original sample, as modified by blunt ending and adapter attachment. In other words, the nucleotide occupying a specified position of a nucleic acid in the sample may be determined to be the consensus of nucleotides occupying that corresponding position in family member sequences. A consensus nucleotide can be determined by methods such as voting or confidence score, to name two non-limiting, exemplary methods. Families can include sequences of one or both strands of a double-stranded nucleic acid. If members of a family include sequences of both strands from a double-stranded nucleic acid, sequences of one strand are converted to their complement for purposes of compiling all sequences to derive consensus nucleotide(s) or sequences. Some families may include only a single member sequence. In this case, this sequence can be taken as the sequence of a nucleic acid in the sample before amplification. Alternatively, families with only a single member sequence can be eliminated from subsequent analysis.

In some embodiments, the results of the systems and methods disclosed herein are used as an input to generate a report. The report may be in a paper format. For example, a report may provide an indication of the presence or absence of a therapeutic nucleic acid construct in a biological sample. In some embodiments, the report may include an indication of the level of the therapeutic nucleic acid construct in a biological sample.

The various steps of the methods disclosed herein, or the steps carried out by the systems disclosed herein, may be carried out at the same or different times, in the same or different geographical locations, e.g. countries, and/or by the same or different people.

### G. Applications

The present methods can be used to detect the presence or absence of a therapeutic nucleic acid construct in a subject. The present methods can be also used for determining or monitoring the efficacy of the treatment by the relative amounts of the therapeutic nucleic acid construct at different time points. In some cases, the amounts of the therapeutic nucleic acid construct can be higher at a second time point compared to a first time point. This can indicate replication of the construct and/or enhanced expression of the construct in the body of the subject. In some cases, the amounts of the therapeutic nucleic acid construct can be lower at the second time point compared to the first time point. This can indicate degradation of the construct in the body of the subject and the subject may be in need of an alternative treatment. In some cases, the amounts of the therapeutic nucleic acid construct can be the same between the time points. This may indicate stable levels of the therapeutic nucleic acid construct in the body. Similarly, the efficacy of different treatments in different cohorts can be determined by the relative amounts of the different therapeutic nucleic acid constructs at one or dissimilar between the time points. This may indicate stability of the construct in the body of the subject. The relative amounts at different time points can be assessed in conjunction with the outcome of the treatment for making recommendations.

In some cases, duration between the time points can be determined by the therapy the subject is undergoing. For example, the subject may need the therapeutic construct only for the duration of the chemotherapy or radiation therapy for improving effectiveness of the therapy. In this case, the therapeutic construct is needed for a specific duration of time and the time points can be spaced to cover the therapy. In some cases, the therapeutic construct may be needed at a specific location in the body, e.g., an organ or a tissue. In this case, the construct can be quantified at both the desired and the non-desired locations at different time points to assess the penetration of the construct into non-desired locations. The present methods can also be used to indicate a drug administered to the subject. The therapeutic nucleic acid construct can be administered in conjunction with a drug, e.g., chemotherapy drug and/or
therapy, for improving outcome of the treatment. The combination of the therapeutic nucleic acid construct with the drug or therapy can yield an enhanced therapeutic effect. In this case, the presence of the therapeutic nucleic acid construct can indicate the presence of the combination drug or therapy. For example, modified oncolytic adenoviruses (e.g., ONYX-015) can be used in combination with radiation and/or temozolomide chemotherapy. Thus, the presence of the oncolytic adenoviruses can be used for inferring the combination therapy the subject may be undergoing.

### EXAMPLES

### EXAMPLE 1: TP53 Expression Vector Detection in a Cancer Subject

A human subject with weight loss and abdominal pain was found to have a tumor in the head of the pancreas and a metastatic liver lesion. Fine-needle aspirate of the tumor confirmed mucinous adenocarcinoma of the pancreas but the sample was insufficient for molecular testing. cfDNA NGS was performed (Guardant Health, CA) to identify genomic alterations (e.g., BRCA, EGFR alterations, ERBB2 amplification).

Guardant360^{®} (G360) is a plasma-based comprehensive cfDNA NGS assay that assesses 73 genes, primarily focused on genomic alterations that are associated with 1) FDA approved targeted therapies, 2) targeted therapies in late stage clinical trials, 3) known predictive or prognostic value, or 4) informative of the presence of cfDNA. Single nucleotide variants (SNVs) are assessed in 73 genes, copy number amplification (CNA) is assessed in 18 genes, insertion deletion variants (indels) are assessed in 23 genes, and fusions are assessed in 6 genes (Table 1).

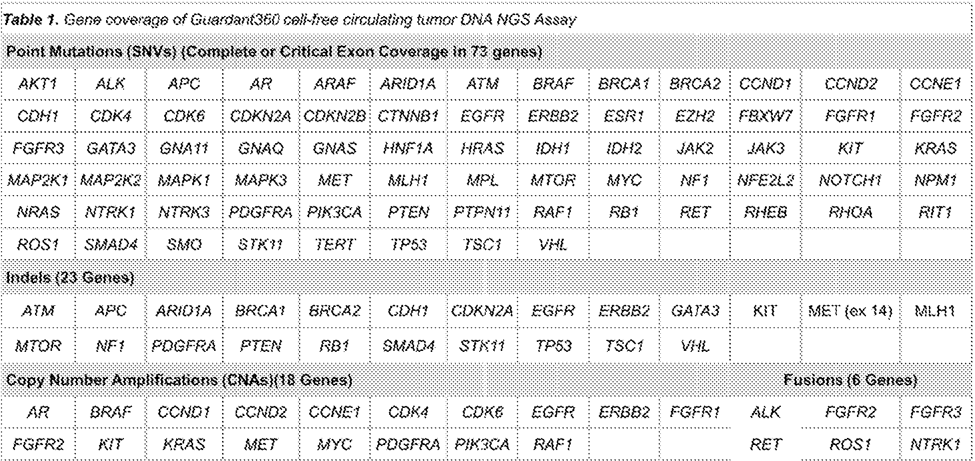

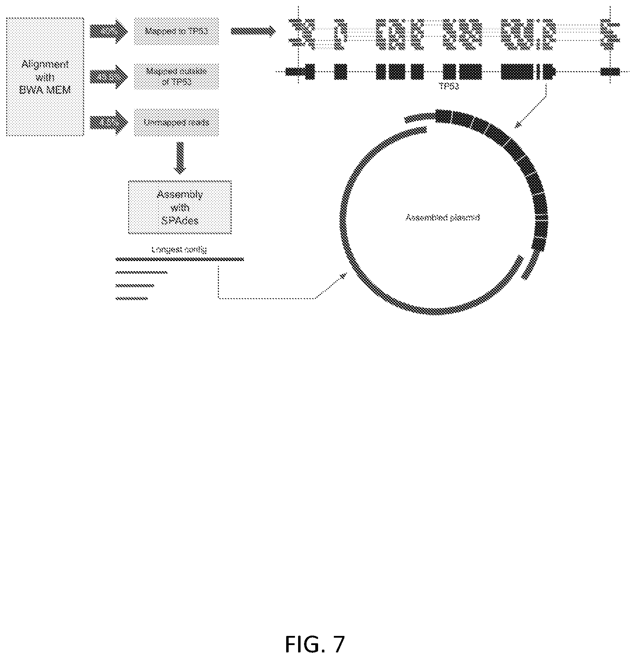

A large (50+) copy number increase in the TP53 gene, which is not part of the G360 reportable region for copy number variants prompted additional follow-up. cfDNA NGS data revealed dramatic over-representation (41% of all reads as compared to the expected 1.5%) of TP53-derived sequences with atypical insert sizes and distribution, read start/stop positions, and post-clipping read lengths. Atypical sequences comprised only TP53 coding sequence, without introns. Unmapped reads were assembled using SPAdes (St. Petersburg Genome Assembler), an iterative short-read genome assembly algorithm, into a 6003 bp circular contig containing the atypical exonic TP53 sequence (FIG. 7). Outside of the TP53 sequence, no sequence of the circular contig mapped to the human genome.

Consultation with the treating physician revealed that the patient progressed on FOLFIRINOX and was subsequently enrolled in a clinical trial utilizing a liposome-encapsulated TP53 expression vector with gemcitabine and nab-paclitaxel. The subject's first experimental treatment was four days prior to cfDNA sample collection. Importantly, over-representation of this synthetic construct obscured native TP53 sequences resulting in a 47% increase in predicted assay limit of detection despite standard performance metrics being within normal ranges. The atypical features of the expression vector reads make it possible to automate detection and quantification, even at significantly lower concentration than described here. The expression vector bears no human genome homology, allowing read identification via mapping. TP53 intron "deletions" were detected by a deletion detection algorithm. Finally, insert length and read start-stop distributions are markedly dissimilar between native and synthetic sequences.

### EXAMPLE 2: Implications of the Vector Presence on Clinical Results

Nucleic acid-based therapies (or NATs) are a unique and previously unrecognized source of interference in liquid biopsies. Therapeutic nucleic acid-specific features may be used to identify and filter such interference using automatable and generalizable bioinformatics techniques (Table 2).

| **Table 2.** *Implications of the vector presence on clinical results* | | | |
|---|---|---|---|
| *Artifact* | *Impact on assay performance* | *Mitigation* | *Role as a potential biomarker* |
| Increased *TP53* coverage | 1. Increased off-target coverage → decreased panel-wide coverage → decreased sensitivity | 1. Flag reportable content failing minimum coverage requirements | Potential sign of NAT presence (limited as increased coverage can be caused by somatic gene amplifications as well) |
| | | 2. Flag and filter content with excess coverage | |
| | 2. Obscured true *TP53* mutations | | |
| | 3. Increased unique molecule count → decreased family size → decreased sensitivity | 3. Flag and filter molecular barcode overcrowding | |
| Absent *TP53* introns | Alignment artifacts at exon-intron junctions → false positive splice site SNV calls | Detection and realignment around exon deletions prior to SNV calling | Potential NAT biomarker (potential interference from processed pseudogenes) |
| Foreign sequence flanking *TP53* | Alignment artifacts at exon-intron junctions → false positive splice site SNV calls | Local reassembly at sites with excess alignment soft-clipping prior to SNV calling | Potential sensitive NAT biomarker, assuming UTRs included in panel |
| Foreign sequence vector backbone | Minimal (miniscule increase in number of unmapped reads) | None needed | Potential NAT biomarker (limited as backbone sequences are captured poorly) |
| Atypical molecule size distribution | Interference with algorithms relying on nucleosome-driven molecule size distribution | None needed | Potential NAT biomarker |

If different versions of a sequence are associated with an accession number at different times, the version associated with the accession number at the effective filing date of this application is meant. The effective filing date means the earlier of the actual filing date or filing date of a priority application referring to the accession number if applicable. Likewise, if different versions of a publication, website or the like are published at different times, the version most recently published at the effective filing date of the application is meant unless otherwise indicated.

## Claims

1. A method of detecting a presence or absence of a therapeutic nucleic acid construct in a biological sample from a test subject at least partially using a computer, the method comprising:
receiving, by the computer, test sequence information comprising sequence reads obtained from cell-free nucleic acid molecules in the biological sample;
removing, by the computer, one or more of the sequence reads that originate from one or more intronic regions and/or from one or more regions spanning exon-intron junctions from the test sequence information to generate filtered test sequence information; or identifying, by the computer, one or more of the sequence reads that originate from one or more regions spanning exon-exon junctions from the test sequence information to generate enriched test sequence information; or identifying, by the computer, at least one set of the sequence reads comprising at least one locus in common with one another, which set comprises a coverage that exceeds a threshold value to thereby identify a candidate set of sequence reads; and,
identifying, by the computer, one or more of the sequence reads in the filtered or the enriched test sequence information, or at least some members of the candidate set of sequence reads, that substantially align with differentiating reference sequence information that originates from the therapeutic nucleic acid construct, thereby detecting the presence or absence of the therapeutic nucleic acid construct in the biological sample from the test subject.

2. The method of claim 1, wherein the method comprises differentiating reference sequence information that originates from a vector portion of the therapeutic nucleic acid construct.

3. The method of claim 1 or claim 2, wherein a plurality of said sequence reads are present in a unimodal size distribution.

4. The method of any one of the preceding claims, wherein the biological sample is selected from the group consisting of: blood, plasma, and serum.

5. The method of any one of the preceding claims, further comprising
generating the test sequence information from the cell-free nucleic acid molecules in the biological sample; and/or
amplifying one or more segments of the cell-free nucleic acid molecules in the biological sample to generate amplified nucleic acid molecules, optionally further comprising sequencing the amplified nucleic acid molecule to generate the test sequence information.

6. The method of any one of the preceding claims,
(i) wherein the test sequence information is obtained from targeted sequences of the cell-free nucleic acid molecules in the biological sample, wherein the targeted sequences are obtained by selectively enriching one or more regions from the cell-free nucleic acid molecules in the biological sample prior to sequencing, the method optionally further comprising amplifying the obtained targeted sequences prior to sequencing;
(ii) further comprising attaching one or more adapters comprising barcodes to the cell-free nucleic acid molecules prior to sequencing; and/or
(iii) wherein the sequencing is selected from the group consisting of: targeted sequencing, intron sequencing, exome sequencing, and whole genome sequencing.

7. The method of any one of the preceding claims, wherein the test subject is a mammalian subject, for example wherein the mammalian subject is a human subject, optionally wherein the test subject has a disease or disorder capable of being treated with the therapeutic nucleic acid construct, for example wherein
(i) the disease is cancer;
(ii) the disorder is a genetic disorder;
(iii) the disease is a viral disease; or
(iv) the disease or disorder is selected from the group consisting of immune deficiency disorders, hemophilia, thalassemia, sickle cell disease, blood disease, chronic granulomatous disorder, congenital blindness, lysosomal storage disease, muscular dystrophy, cancer, neurodegenerative disease, viral infections, bacterial infections, epidermolysis bullosa, heart disease, fat metabolism disorder, and diabetes, or a combination thereof.

8. The method of any one of the preceding claims, wherein the therapeutic nucleic acid construct
(i) is a construct used in a DNA-based therapy, for example wherein the DNA-based therapeutic construct is selected from the group consisting of plasmids, aptamers, DNAzymes, antisense oligonucleotides, viral vectors, and antigene oligonucleotides;
(ii) comprises a natural nucleotide sequence, a variant of the natural nucleotide sequence or a synthetic nucleotide sequence or combinations thereof;
(iii) is part of a chimeric antigen receptor (CAR) T-cell therapy;
(iv) is part of a CRISPR/Cas therapy; or
(v) comprises a portion of a human adenovirus nucleic acid sequence.

9. The method of any one of the preceding claims, wherein the therapeutic nucleic acid construct is a construct used in an RNA-based therapy, for example wherein the RNA-based therapeutic nucleic acid construct is selected from the group consisting of aptamers, RNA decoys, antisense RNA, ribozymes, small interfering RNAs, and microRNA.

10. The method of any one of the preceding claims, wherein the biological sample comprises fragments of the therapeutic nucleic acid construct, the method optionally further comprising selectively enriching for a target sequence of the therapeutic nucleic acid construct, for example wherein the target sequence comprises
(i) a natural nucleotide sequence, a variant of the natural nucleotide sequence or a synthetic nucleotide sequence or combinations thereof; or
(ii) a gene or a variant thereof selected from the group consisting of *TP53, HBB, RPE65, and B-domain deleted factor VIII gene, DMD, UL123, APOB, SMN1, SMN2, ICAM-1, TLR9, IRS-1, VEGF, PDGFA, PDGFB, PDGFC, PDGFD,* and *TTR.*

11. The method of any one of the preceding claims, further comprising using results of the method as an input to generate a report, for example wherein the report provides an indication of the presence or absence of the therapeutic nucleic acid construct in the biological sample from the test subject.

12. A system comprising means for carrying out the method of claim 1.

13. A computer program comprising instructions which, when the program is executed by a computer, cause the computer to carry out the method of claim 1.

14. A computer-readable storage medium comprising instructions which, when executed by a computer, cause the computer to carry out the method of claim 1.

## Patentansprüche

1. Verfahren zum Nachweisen einer Anwesenheit oder Abwesenheit eines therapeutischen Nukleinsäurekonstrukts in einer biologischen Probe von einem Testindividuum zumindest teilweise unter Verwendung eines Computers, wobei das Verfahren Folgendes umfasst:
Empfangen, durch den Computer, von Test-Sequenzinformationen, die Sequenzablesungen umfassen, die von zellfreien Nukleinsäuremolekülen in der biologischen Probe erhalten wurden;
Entfernen, durch den Computer, einer oder mehrerer der Sequenzablesungen, die von einer oder mehreren Intronregionen und/oder von einer oder mehreren Regionen stammen, die Exon-Intron-Verbindungen überspannen, aus den Test-Sequenzinformationen, um gefilterte Test-Sequenzinformationen zu erzeugen; oder Identifizieren, durch den Computer, einer oder mehrerer der Sequenzablesungen, die von einer oder mehreren Regionen stammen, die Exon-Exon-Verbindungen überspannen, aus den Test-Sequenzinformationen, um angereicherte Test-Sequenzinformationen zu erzeugen; oder Identifizieren, durch den Computer, mindestens eines Satzes der Sequenzablesungen, die mindestens einen gemeinsamen Locus umfassen, wobei der Satz eine Abdeckung umfasst, die einen Schwellenwert überschreitet, um dadurch einen Kandidatensatz von Sequenzablesungen zu identifizieren; und
Identifizieren, durch den Computer, einer oder mehrerer der Sequenzablesungen in den gefilterten oder den angereicherten Test-Sequenzinformationen oder mindestens einiger Mitglieder des Kandidatensatzes von Sequenzablesungen, die im Wesentlichen mit unterscheidenden Referenz-Sequenzinformationen alignieren, die von dem therapeutischen Nukleinsäurekonstrukt stammen, wodurch die Anwesenheit oder Abwesenheit des therapeutischen Nukleinsäurekonstrukts in der biologischen Probe von dem Testindividuum nachgewiesen wird.

2. Verfahren nach Anspruch 1, wobei das Verfahren unterscheidende Referenz-Sequenzinformationen umfasst, die von einem Vektorabschnitt des therapeutischen Nukleinsäurekonstrukts stammen.

3. Verfahren nach Anspruch 1 oder Anspruch 2, wobei eine Mehrzahl der Sequenzablesungen in einer unimodalen Größenverteilung vorliegt.

4. Verfahren nach einem der vorhergehenden Ansprüche, wobei die biologische Probe aus der Gruppe ausgewählt ist, bestehend aus: Blut, Plasma und Serum.

5. Verfahren nach einem der vorhergehenden Ansprüche, ferner umfassend das Erzeugen der Test-Sequenzinformationen aus den zellfreien Nukleinsäuremolekülen in der biologischen Probe und/oder Amplifizieren eines oder mehrerer Segmente der zellfreien Nukleinsäuremoleküle in der biologischen Probe, um amplifizierte Nukleinsäuremoleküle zu erzeugen, optional ferner umfassend Sequenzieren des amplifizierten Nukleinsäuremoleküls, um die Test-Sequenzinformationen zu erzeugen.

6. Verfahren nach einem der vorhergehenden Ansprüche,
(i) wobei die Test-Sequenzinformationen von Zielsequenzen der zellfreien Nukleinsäuremoleküle in der biologischen Probe erhalten werden, wobei die Zielsequenzen durch selektives Anreichern einer oder mehrerer Regionen aus den zellfreien Nukleinsäuremolekülen in der biologischen Probe vor dem Sequenzieren erhalten werden, wobei das Verfahren optional ferner das Amplifizieren der erhaltenen Zielsequenzen vor dem Sequenzieren umfasst,
(ii) ferner umfassend das Anheften eines oder mehrerer Adapter, die Barcodes umfassen, an die zellfreien Nukleinsäuremoleküle vor dem Sequenzieren, und/oder
(iii) wobei das Sequenzieren aus der Gruppe ausgewählt ist, bestehend aus: gezielter Sequenzierung, Intron-Sequenzierung, Exom-Sequenzierung und Ganz-Genom-Sequenzierung.

7. Verfahren nach einem der vorhergehenden Ansprüche, wobei das Testindividuum ein Säugerindividuum ist, wobei zum Beispiel das Säugerindividuum ein menschliches Individuum ist, wobei optional das Testindividuum eine Krankheit oder Störung hat, die mit dem therapeutischen Nukleinsäurekonstrukt behandelt werden kann, wobei zum Beispiel
(i) die Krankheit Krebs ist,
(ii) die Störung eine genetische Störung ist,
(iii) die Krankheit eine Viruserkrankung ist oder
(iv) die Krankheit oder Störung aus der Gruppe ausgewählt ist, bestehend aus Immundefizienzstörungen, Hämophilie, Thalassämie, Sichelzellkrankheit, Blutkrankheit, chronischer granulomatöser Störung, angeborener Blindheit, lysosomaler Speicherkrankheit, Muskeldystrophie, Krebs, neurodegenerativer Krankheit, Virusinfektionen, bakteriellen Infektionen, Epidermolysis bullosa, Herzkrankheit, Fettstoffwechselstörung und Diabetes oder einer Kombination davon.

8. Verfahren nach einem der vorhergehenden Ansprüche, wobei das therapeutische Nukleinsäurekonstrukt
(i) ein bei einer DNA-basierten Therapie verwendetes Konstrukt ist, wobei zum Beispiel das DNA-basierte therapeutische Konstrukt aus der Gruppe ausgewählt ist, bestehend aus Plasmiden, Aptameren, DNAzymen, Antisense-Oligonukleotiden, Virusvektoren und Antigen-Oligonukleotiden,
(ii) eine natürliche Nukleotidsequenz, eine Variante der natürlichen Nukleotidsequenz oder eine synthetische Nukleotidsequenz oder Kombinationen davon umfasst,
(iii) Teil einer chimärer-Antigen-Rezeptor(CAR)-T-Zell-Therapie ist,
(iv) Teil einer CRISPR/CAS-Therapie ist oder
(v) einen Abschnitt einer Nukleinsäuresequenz eines menschlichen Adenovirus umfasst.

9. Verfahren nach einem der vorhergehenden Ansprüche, wobei das therapeutische Nukleinsäurekonstrukt ein bei einer RNA-basierten Therapie verwendetes Konstrukt ist, wobei zum Beispiel das RNA-basierte therapeutische Nukleinsäurekonstrukt aus der Gruppe ausgewählt ist, bestehend aus Aptameren, RNA-Decoys, Antisense-RNA, Ribozymen, kleinen interferierenden RNAs und mikroRNA.

10. Verfahren nach einem der vorhergehenden Ansprüche, wobei die biologische Probe Fragmente des therapeutischen Nukleinsäurekonstrukts umfasst, wobei das Verfahren optional ferner das selektive Anreichern einer Zielsequenz des therapeutischen Nukleinsäurekonstrukts umfasst, wobei zum Beispiel die Zielsequenz Folgendes umfasst:
(i) eine natürliche Nukleotidsequenz, eine Variante der natürlichen Nukleotidsequenz oder eine synthetische Nukleotidsequenz oder Kombinationen davon oder
(ii) ein Gen oder eine Variante davon, ausgewählt aus der Gruppe, bestehend aus TP53-, HBB-, RPE65- und B-Domänendeletiertem Faktor VIII-Gen, DMD, UL123, APOB, SMN1, SMN2, ICAM-1, TLR9, IRS-1, VEGF, PDGFA, PDGFB, PDGFC, PDGFD UND TTR.

11. Verfahren nach einem der vorhergehenden Ansprüche, ferner umfassend das Verwenden von Ergebnissen des Verfahrens als Eingabe zum Erzeugen eines Berichts, wobei zum Beispiel der Bericht einen Hinweis auf die Anwesenheit oder Abwesenheit des therapeutischen Nukleinsäurekonstrukts in der biologischen Probe von dem Testindividuum liefert.

12. System, umfassend Mittel zum Durchführen des Verfahrens nach Anspruch 1.

13. Computerprogramm, umfassend Anweisungen, die, wenn das Programm von einem Computer ausgeführt wird, den Computer dazu veranlassen, das Verfahren nach Anspruch 1 durchzuführen.

14. Computerlesbares Speichermedium, umfassend Anweisungen, die, wenn sie von einem Computer ausgeführt werden, den Computer dazu veranlassen, das Verfahren nach Anspruch 1 durchzuführen.

## Revendications

1. Procédé de détection de la présence ou de l'absence d'une construction d'acide nucléique thérapeutique dans un échantillon biologique d'un sujet d'essai, au moins partiellement au moyen d'un ordinateur, le procédé comprenant :
la réception, par l'ordinateur, d'informations de séquence d'essai comprenant des lectures de séquence obtenues à partir de molécules d'acide nucléique acellulaires dans l'échantillon biologique ;
le retrait, par l'ordinateur, d'une ou plusieurs des lectures de séquence provenant d'une ou plusieurs régions introniques et/ou d'une ou plusieurs régions couvrant des jonctions exon-intron des informations de séquence d'essai pour générer des informations de séquence d'essai filtrées ; ou l'identification, par l'ordinateur, d'une ou plusieurs lectures de séquence qui proviennent d'une ou plusieurs régions couvrant des jonctions exon-exon à partir des informations de séquence d'essai pour générer des informations de séquence d'essai enrichies ; ou
l'identification, par l'ordinateur, d'au moins un ensemble des lectures de séquence ayant au moins un locus en commun, ledit ensemble comprenant une couverture dépassant une valeur seuil pour identifier ainsi un ensemble candidat de lectures de séquence ; et,
l'identification, par l'ordinateur, d'une ou plusieurs des lectures de séquence dans les informations de séquence d'essai filtrées ou enrichies, ou d'au moins certains éléments de l'ensemble candidat de lectures de séquence, qui s'alignent sensiblement avec les informations de séquence de référence distinctives qui proviennent de la construction d'acide nucléique thérapeutique, détectant ainsi la présence ou l'absence de la construction d'acide nucléique thérapeutique dans l'échantillon biologique provenant du sujet d'essai.

2. Procédé selon la revendication 1, le procédé comprenant des informations de séquence de référence distinctives qui proviennent d'une partie de vecteur de la construction d'acide nucléique thérapeutique.

3. Procédé selon la revendication 1 ou la revendication 2, dans lequel une pluralité desdites lectures de séquence est présente dans une distribution de taille unimodale.

4. Procédé selon l'une quelconque des revendications précédentes, dans lequel l'échantillon biologique est choisi dans le groupe constitué par du sang total, du plasma et du sérum.

5. Procédé selon l'une quelconque des revendications précédentes, comprenant en outre la génération des informations de séquence d'essai à partir des molécules d'acide nucléique acellulaires dans l'échantillon biologique ; et/ou
l'amplification d'un ou plusieurs segments des molécules d'acide nucléique acellulaires dans l'échantillon biologique pour générer des molécules d'acide nucléique amplifiées, facultativement, comprenant en outre le séquençage de la molécule d'acide nucléique amplifiée pour générer les informations de séquence d'essai.

6. Procédé selon l'une quelconque des revendications précédentes,
(i) dans lequel les informations de séquence d'essai sont obtenues à partir de séquences ciblées des molécules d'acide nucléique acellulaires dans l'échantillon biologique, les séquences ciblées étant obtenues par enrichissement sélectif d'une ou plusieurs régions à partir des molécules d'acide nucléique acellulaires dans l'échantillon biologique avant le séquençage, le procédé comprenant facultativement en outre l'amplification des séquences ciblées obtenues avant le séquençage ;
(ii) comprenant en outre la liaison d'un ou plusieurs adaptateurs comprenant des codes-barres aux molécules d'acide nucléique acellulaires avant le séquençage ; et/ou
(iii) dans lequel le séquençage est choisi dans le groupe constitué par : un séquençage ciblé, un séquençage d'intron, un séquençage de l'exome, et un séquençage du génome entier.

7. Procédé de l'une quelconque des revendications précédentes, dans lequel le sujet d'essai est un sujet mammifère, par exemple dans lequel le sujet mammifère est un sujet humain, facultativement, dans lequel le sujet d'essai est atteint d'une maladie ou d'un trouble pouvant être traité au moyen de la construction d'acide nucléique thérapeutique, par exemple dans lequel
(i) la maladie est un cancer ;
(ii) le trouble est un trouble génétique ;
(iii) la maladie est une maladie virale ; ou
(iv) la maladie ou le trouble est choisi dans le groupe constitué par les troubles de l'immunodéficience, l'hémophilie, la thalassémie, la drépanocytose, une hémopathie, un trouble granulomateux chronique, la cécité congénitale, une maladie de surcharge lysosomale, une dystrophie musculaire, un cancer, une maladie neurodégénérative, les infections virales, les infections bactériennes, l'épidermolyse bulleuse, une maladie cardiaque, un trouble du métabolisme lipidique, et le diabète, ou une combinaison de ceux-ci.

8. Procédé selon l'une quelconque des revendications précédentes, dans lequel la construction d'acide nucléique thérapeutique
(i) est une construction utilisée dans une thérapie à base d'ADN, par exemple dans lequel la construction thérapeutique à base d'ADN est choisie dans le groupe constitué par des plasmides, des aptamères, des ADNzymes, des oligonucléotides antisens, des vecteurs viraux et des oligonucléotides antigéniques ;
(ii) comprend une séquence nucléotidique naturelle, un variant de la séquence nucléotidique naturelle ou une séquence nucléotidique synthétique ou des combinaisons de celles-ci ;
(iii) fait partie d'une thérapie par lymphocytes T à récepteur antigénique chimérique (CAR) ;
(iv) fait partie d'une thérapie CRISPR/Cas ; ou
(v) comprend une partie d'une séquence d'acide nucléique d'adénovirus humain.

9. Procédé selon l'une quelconque des revendications précédentes, dans lequel la construction d'acide nucléique thérapeutique est une construction utilisée dans une thérapie à base d'ARN, par exemple dans lequel la construction d'acide nucléique thérapeutique à base d'ARN est choisie dans le groupe constitué par des aptamères, des ARN leurres, un ARN antisens, des ribozymes, des petits ARN interférents et des microARN.

10. Procédé selon l'une quelconque des revendications précédentes, dans lequel l'échantillon biologique comprend des fragments de la construction d'acide nucléique thérapeutique, le procédé comprenant facultativement en outre l'enrichissement sélectif d'une séquence cible de la construction d'acide nucléique thérapeutique, par exemple dans lequel la séquence cible comprend
(i) une séquence nucléotidique naturelle, un variant de la séquence nucléotidique naturelle ou une séquence nucléotidique synthétique ou des combinaisons de celles-ci ; ou
(ii) un gène ou un variant de celui-ci choisi dans le groupe constitué par TP53, HBB, RPE65, et le gène de facteur VIII dans lequel le domaine B a été délété, DMD, UL123, APOB, SMN1, SMN2, ICAM-1, TLR9, IRS-1, VEGF, PDGFA, PDGFB, PDGFC, PDGFD et TTR.

11. Procédé selon l'une quelconque des revendications précédentes, comprenant en outre l'utilisation des résultats du procédé en entrée pour générer un rapport, par exemple dans lequel le rapport fournit une indication de la présence ou de l'absence de la construction d'acide nucléique thérapeutique dans l'échantillon biologique provenant du sujet d'essai.

12. Système comprenant des moyens pour mettre en œuvre le procédé selon la revendication 1.

13. Programme informatique comprenant des instructions qui, lorsque le programme est exécuté par un ordinateur, amènent l'ordinateur à réaliser le procédé selon la revendication 1.

14. Support de stockage lisible par ordinateur comprenant des instructions qui, lorsqu'elles sont exécutées par un ordinateur, amènent l'ordinateur à réaliser le procédé selon la revendication 1.
